# EUROPEAN PATENT APPLICATION

(11) **EP 4 098 745 A1**
(43) Date of publication of application: **07.12.2022**
(21) Application number: 21177135.7
(22) Date of filing: 01.06.2021
(51) Int. Cl.: C12N 15/113

(54) **ANTISENSE OLIGONUCLEOTIDES (ASO) FOR EFFICIENT AND PRECISE RNA EDITING WITH ENDOGENOUS ADENOSINE DEAMINASE ACTING ON RNA (ADAR)**

(71) Applicant: Eberhard-Karls-Universität Tübingen, 72074 Tübingen (DE)
(72) Inventor: Stafforst, Thorsten, 72076 Tübingen (DE); Merkle, Tobias, 2100 Kopenhagen (DK); Wettengel, Jacqueline, 72074 Tübingen (DE)
(74) Representative: Gill Jennings & Every LLP

(57) **Abstract**

The present invention relates to a chemically modified oligoribonucleotide for use in site-directed A-to-I editing of a target RNA inside a cell with endogenous ADAR, comprising a sequence with a length from 25 to 80 nucleotides, capable of binding to a target sequence in the target RNA, comprising a Central Base Triplet of 3 nucleotides with the central nucleotide opposite to the target adenosine in the target RNA which is to be edited to an inosine, having
a) at least 90% of the pyrimidine nucleosides outside the Central Base Triplet are chemically modified, either at the 2' position of the sugar moiety or as deoxyribonucleosides, or a combination thereof,
b) no more than 6 consecutive nucleosides are chemically modified with 2'-O-methyl at the 2' position of the sugar moiety,
c) at least two of the three nucleosides of the Central Base Triplet are chemically modified at the 2' position of the sugar moiety, or are deoxyribonucleosides.

## Description

The present invention concerns processes and chemically modified nucleic acids for use of site-directed editing of a target RNA. In view of the tremendous progress made by molecular biology it is possible to alter the genetic information of cells. While the editing of DNA usually leads to stable modification of the genetic information of the cells it is sometimes interesting not to change the DNA but to change the genetic information in the (m)RNA. A major advantage of editing (m)RNA over DNA is on the one hand the dose-dependency of the editing yield and on the other hand the reversibility of the treatment. By regulating the concentration of the chemically modified nucleic acid in the cells where the target RNA shall be edited a dependency on the editing yield and thus the amount of modified protein after translation of the target RNA can be achieved. On the other hand, the treatment is reversible since the editing of the target RNA is halted when the chemically modified nucleic acid is no longer present in the relevant cell, and the edited RNA is replaced by newly transcribed unedited RNA.

Editing of RNA molecules according to the invention is mediated by enzymes belonging to the family of adenosine deaminases acting on RNA (ADARs). ADARs are members of an enzyme family that catalyze the deamination of adenosine (A) to inosine (I) in double-stranded RNA (A-to-I RNA editing). In the course of this enzymatically catalyzed reaction adenosine is changed via a hydrated intermediate to inosine. While guanosine can form three hydrogen bonds to the complementary base cytidine, inosine can form only two hydrogen bonds to cytidine. The translational machinery reads inosine as a guanosine. Therefore, ADARs have the effect of introducing a functional adenosine to guanosine mutation on the RNA level.

A requirement that a deaminase belonging to the ADAR family can act on RNA, in particular mRNA, is that a double strand is formed. Therefore, it is required to provide a complementary nucleic acid (in the following: oligonucleotide or oligoribonucleotide) which can form a double-stranded molecule on which the ADAR can act.

The present invention discloses chemically modified nucleic acids which can cause a functional change from an adenosine (A) to a guanosine (G). Depending on the sequence of the RNA such a mutation can have dramatic effects. The mutation may either cure point mutations which have deleterious effects of the protein encoded by the mRNA or other amino acids may be incorporated in the translated protein by substitution. Strong effects can be observed, however, when stop codons (UAA, UAG, UGA) are edited or splice sites.

A substantial advantage of the chemically modified oligonucleotides of the present invention is that such off-target edits are reversible and the danger of devastating side effects is less likely. Moreover, therapy can be stopped and reverted if necessary. Due to the better safety profile the temporary and limited manipulation of human genetic information at the RNA level may become broadly applicable and may be expanded to medical indications whereby genome editing on the DNA level may be dangerous due to unforeseeable and irreversible side effects.

There are several ADAR enzymes expressed across human tissues which enable the conversion of adenosine to inosine, which in turn is biochemically read in translation as guanosine. Several ADARs are known in the art. ADAR has been found in *Xenopus levis* but also in human and murine cells. While all three human ADARs share a common C-terminal deaminase domain, only ADAR 1 and ADAR 2 revealed to be catalytically active. ADARs share a common functional domain which is the double-stranded RNA binding domain (dsRBD). While ADAR 1 contains three, ADAR 2 and ADAR 3 share only two dsRBDs. For ADAR 1 two isoforms are known. The short constitutively expressed 110 kDa ADAR 1 is the p110 isoform whereas the longer 150 kDa ADAR 1 is the p150 isoform, which is expressed from an alternative interferon inducible promoter. According to the present knowledge, ADAR 2 predominantly edits coding sites in the brain. The ADAR 1 is the major enzyme for editing non-coding sites.

For an efficient editing of RNA it is necessary that the ADAR is directed to specific target sites on the mRNA transcript. Previous attempts in the prior art utilized a specific, loop-hairpin structured ADAR recruiting moiety derived from natural, cis-acting ADAR recruiting sequences to direct the deaminase activity of ADARs to specific sites, thereby bringing the deaminase activity of ADAR to the correct position on the mRNA molecule to be edited. Such artificial nucleic acids for site-directed RNA editing are disclosed in WO 2020/001793. The artificial nucleic acid disclosed in the prior art comprises a targeting sequence, which in turn comprises a nucleic acid sequence complementary or at least partially complementary to a target sequence in the target RNA and a recruiting moiety for recruiting a deaminase.

The chemically modified nucleic acids according to the present invention differ from the nucleic acid oligonucleotides disclosed therein insofar that they do not have a loop-hairpin structured recruiting moiety specifically for recruiting a deaminase. The chemically modified nucleic acids of the present invention use another strategy than the constructs known from the prior art. It is well-known that RNAs are highly instable due to the ubiquitous presence of different RNA digesting enzymes, in particular RNase A and H. Editing of RNA with the constructs of prior art is only achieved by recruiting the deaminase with the help of the recruiting moiety. The recruiting moiety guides the deaminase to the desired site of action, namely the target adenosine which shall be converted to an inosine, but functionally a guanosine.

Contrary thereto the chemically modified nucleic acids of the present invention do not necessarily have a loop-hairpin structured recruiting moiety for a deaminase. Instead, the chemically modified nucleic acids of the present invention form an RNA duplex to which the ADAR enzyme adheres, whereby the editing efficiency is increased. The latter is achieved by using chemically modified nucleic acids of a specific optimal chemical modification pattern over its whole length. It is an important aspect of the present invention that the chemical modification of the ASO is not limited to the central triplet but it extends over the flanks adjacent to the central triplet.

The central triplet of the target RNA sequence comprises an adenosine flanked by one nucleotide on both sides, and will be further referred to as the Central Base Triplet. The sequence complementary to the Central Base Triplet in the chemically modified oligonucleotide of the present invention is important with regard to its specific chemical modification. In order to enable a functional change on the translational level of the mRNA (editing) it is required that the oligonucleotide according to the invention allows the editing of the mRNA. It is essential that the oligonucleotides according to the invention are on the one hand sufficiently stabilized against degradation (caused e.g. by RNase) which can be achieved by chemical modification of the oligonucleotide, in particular by modification of the sugar moieties of the oligonucleotide.

On the other hand, the chemical modification must allow the editing of the RNA molecule. If the chemical modification of the oligonucleotide is too extensive, the efficacy of editing is reduced to an unacceptable level. Therefore, the modification of the oligonucleotide must follow the guidelines as described herein in order to obtain an optimal editing efficacy.

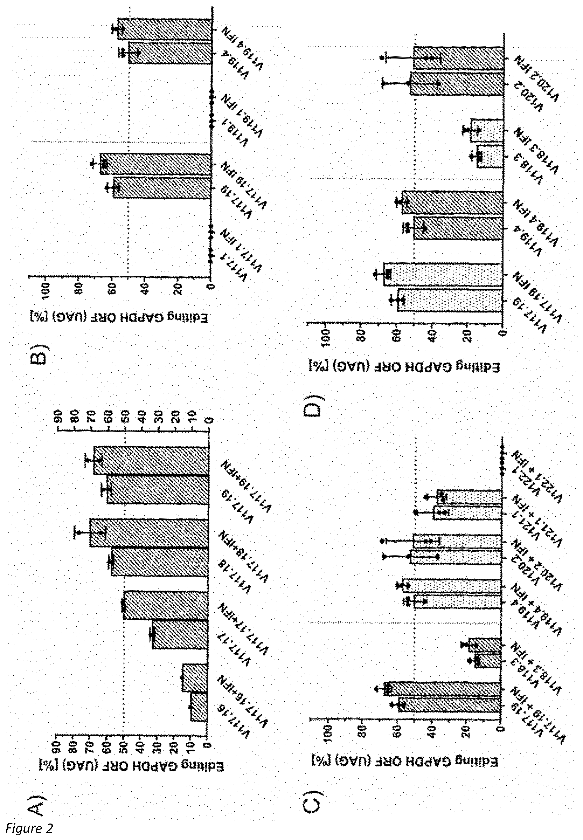

EP 3 507 366 discloses chemically modified single-stranded RNA-editing oligonucleotides for the deamination of a target adenosine by an ADAR enzyme whereby the central base triplet of 3 sequential nucleotides comprises a sugar modification and/or a base modification. The flanking regions, in all embodiments (Fig. 2 of said prior art), are uniformly modified with blocks of 2'-O-methylation at the ribose units plus a small number of additional terminal phosphorothioate linkages.

However, it was found that such uniform and block-wise 2'-O-methyl modification of the nucleic acids as used in the prior art above leads to a strong loss of editing activity with natural ADAR enzymes at their endogenous expression levels. This is in accordance with a negative effect of bulky 2'-modifications on the binding of double-stranded RNA binding domains to dsRNA substrates. Specifically, we describe in our invention that 2'-F and mixtures of 2'-F and 2'-OMe are particularly well accepted and still have a good stabilizing effect against nuclease digestion when they are placed at all pyrimidine bases on the nucleic acids. However, in the Central Base Triplet 2'-F and in particular 2'-O-methylation had a strongly negative effect on editing yield, which is in accordance to literature. However, we found deoxyribose at all three positions of the Central Base Triplet well tolerated and giving substantial stabilization against nuclease digestion. In a preferred embodiment of the present invention the three sugar units of the oligonucleotide complementary to the control triplet are desoxyribose units.

The chemically modified nucleic acids are further characterized in that they are suitable for use in site-directed editing of a target mRNA. The chemically modified nucleic acids comprise a sequence, which is completely complementary to a target sequence in the target mRNA with the exception of the central nucleotide of the Central Base Triplet, which is opposite to the target adenosine. The central nucleotide of the Central Base Triplet is typically a cytosine or a derivative thereof, but can also be a nucleobase analogue, typically built on an N-heterocyclic compound, and replaces the normally complementary thymidine or uracil and usually improves editing site recognition by ADAR. By action of the adenosine deaminase, the target adenosine is functionally changed to a guanosine post-transcriptionally. Therefore, the sequence of the nucleotides is always complementary to the target region of the mRNA with the one exception as previously described to improve the recognition of the targeted adenosine by ADAR within the dsRNA formed when the administered oligonucleotide hybridizes with the target RNA. Important is furthermore the pattern of the modification of the oligonucleotide, in particular of the sugar moieties and the linkages there between.

The principle of the present invention is based on the fact that the chemically modified nucleic acids must be stable for a sufficient period of time in order to allow the editing of the mRNA. Normally, RNA molecules are degraded very quickly in the cells. Therefore, the nucleic acids are chemically modified, whereby the modification must occur to such an extent that the chemically modified nucleic acids survive for a sufficient time span in the cell, and the modifications simultaneously do not hinder recognition by ADAR. The modification of the oligonucleotides relates to the sugar moiety of the nucleotide. RNA bases are the unmodified moieties. The preferred modifications which stabilize the oligonucleotide are deoxy-ribose moieties or RNA bases with 2'-O-methyl or 2'-F modifications at the ribose moiety. Another modification is the replacement of the phosphate bond between the sugar moieties by a phosphorothioate bond.

In the course of the invention it has been found that the artificial nucleic acid (oligonucleotide) has a length of 15 to 80 nucleotides, preferably 25 to 65 nucleotides, more preferably 30-60 nucleotides. Nucleic acids of such length are designated as oligonucleotides in the present application.

The chemically modified nucleic acids (oligonucleotides) according to the invention have a sequence which is complementary to the corresponding sequence in the target mRNA with a complementarity of nearly 100%. In some embodiments the complementarity of the chemically modified oligonucleotides to the corresponding sequence in the target mRNA is at least 85% complementarity. While full complementarity is optimal for the hybridization process, natural ADAR substrates often contain a small number of mismatches and/or bulges, which assist the editing by allowing structural perturbations of the double-stranded substrate to improve substrate recognition by the double-strand RNA binding domain or inside the active site of the deaminase.

In chemically modified nucleic acids (oligonucleotides) according to the invention at least 90% of the pyrimidine nucleosides, preferably 95% and even more preferred 100% are chemically modified at the 2'-position. Outside the Central Base Triplet, the modification is preferably a 2'-alkyl, more preferred a 2'-O-methyl, or 2'-O-ethyl, a 2'-amino or a 2'-halo, more preferred a 2'-fluoro modification. Inside the Central Base Triplet, at least two, more preferably all nucleobases, pyrimidines and purines, are modified with 2'-OMe, or 2'-F, or more preferred with 2'-deoxy. In an embodiment of the invention a terminal block at both termini (5'/3') is present, preferably at each end with three 2'-OMe/PS nucleotides or 2'-MOE/PS.

In a particularly preferred embodiment the pyrimidine nucleosides outside the Central Base Triplet are modified with a mixture of 2'-fluoro and 2'-O-methyl substituents in any stoichiometry, preferably 20:80-80:20, more preferably 70:30-30:70, even more preferably 60:40-40:60. If 2'-F and 2'-O-methyl modifications are mixed, not more than 6, preferably not more then 5, even more preferably not more then 4, 2'-O-methyl modifications should be combined into one block.

In the Central Base Triplet of the chemically modified nucleic acids according to an embodiment of the invention there is a nucleoside carrying an N-heterocyclic base, a pyridine or pyrimidine derivative, more preferably a cytosine nucleoside or a derivative thereof, which is opposite of the target adenosine in the target (m)RNA. In a particularly preferred embodiment this nucleoside and the 5' and 3'-singular neighbouring nucleotides comprise at least one modified nucleoside, more preferred two modified nucleosides, even more preferred three modified nucleosides, having a substituent at the 2' carbon atom whereby the substituent is either 2'-fluoro or 2'-O-methyl, or even more preferred 2'-deoxy.

When a 5'-CAN codon (targeted A underlined, N = any nucleobase) is targeted in a target (m)RNA, then the Central Base Triplet of the chemically modified nucleic acid according to the invention contains a 2'-deoxy-inosine or any nucleotide harbouring a hypoxanthine nucleobase or a derivative thereof, which pairs with the cytosine base 5'-adjacent to the targeted adenosine. Preferably, the 2'-deoxy-inosine is placed in a Central Base Triplet containing two, more preferably three 2'-deoxynucleotides.

Since the chemically modified nucleic acids according to the present invention show increased stability against degradation and an optimal chemical modification pattern to bind ADARs, the nucleic acids according to the present invention preferably do not have a specific loop-hairpin-structured recruiting moiety which attracts the deaminase.

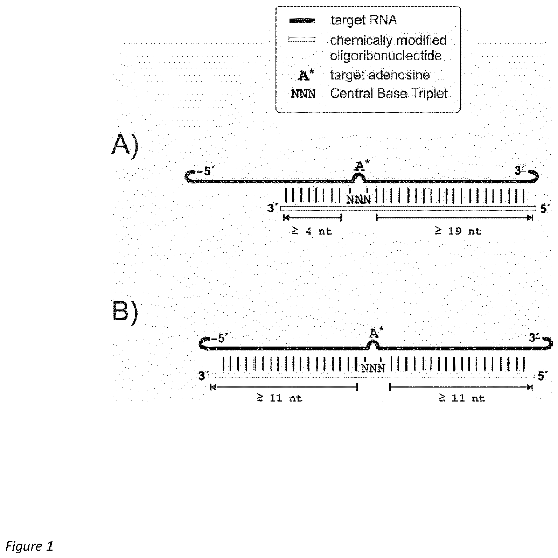

In one embodiment of the present invention the chemically modified nucleic acids are symmetrical, which means that the two nucleotide sequences adjacent to the Central Base Triplet have the same length. When the oligonucleotide has for example 59 nucleotides there are 28 nucleotides on each side of the Central Base Triplet. A schematic structure of the symmetrical embodiment of the present invention is depicted in Figure 1B.

In another embodiment the nucleic acids according to the present invention are not symmetrical which means that the two sequences flanking the Central Base Triplet have different lengths. The asymmetric design enables a more flexible use of the sequence space around the target. Furthermore, it was found that the asymmetric design can enhance editing yields in short sequences of the nucleic acid, e.g. 45 nt, compared to the symmetric design, provided that the nucleic acid is shortened at the correct terminus. Preferably, the flanking sequence 5' to the Central Base Triplet is longer than the flanking sequence 3' in asymmetric embodiments. Preferred embodiments comprise at least 4 nt, more preferred at least 9 nt at the 3' flanking sequence, and comprise at least 19 nt, more preferably at least 28 nt, most preferably at least 33 nt at the 5' flanking sequence. A schematic structure of the asymmetrical embodiment of the present invention is depicted in Figure 1A.

In a preferred embodiment the nucleic acids according to the present invention are linked via phosphorothioate linkages to a percentage of at least 30%, more preferably 40%, even more preferably 50%, most preferably more than 60%. Preferably, at least 10, more preferably at least 15, most preferably 20 or more nucleoside linkages are placed with little discontinuity, more preferably without any discontinuity, starting from a terminus (5' or 3') of the nucleic acid. In another embodiment of the invention said blocks of preferably continuous phosphorothioate linkages are placed on both flanks of the nucleic acid starting from both termini (5' and 3').

In another preferred embodiment modifications of the sugar moiety are combined with phosphorothioate linkages.

The chemically modified nucleic acids according to the present invention are substantially more stable against degradation usually effected by RNases which in turn allows them to be longer present in the cells wherein the (m)RNA should be edited. Without wishing to be bound to a theory, it is assumed that - since the lifetime of the chemically modified nucleic acids is increased in the environment of the cells - no recruiting moiety for recruiting the deaminase is required, because the ADARs can act on the mRNA due to the longer stability of the double strand.

Biological reactions are frequently time-dependent. There is a large variety of different RNA molecules in cells of vertebrates which are subject to a permanent and quick turnover. RNA molecules are frequently degraded by different RNases. Therefore, the use of RNA molecules for therapeutic purposes is frequently limited by the rapid degradation of the RNA molecules. Since the situation in vivo is usually different from the situation in vitro, where test systems with cell cultures are used, the stability of the molecules used for therapeutic purposes may be decisive for the success of the treatment.

The chemically modified nucleic acid molecules according to the invention provide a good balance of editing capability and sufficient stability in the cells whereby even the condition in the endosome can be tolerated. The chemically modified oligonucleotides according to the present invention are furthermore capable of gymnotic uptake and show an editing efficiency which is acceptable.

Best results with the chemically modified nucleic acids according to the invention can be achieved when preferably several, at least two and more preferred at least three of the following features are realized in the oligonucleotide, namely:
- high phosphorothioate content (at least 30%) placed in the regions flanking the Central Base Triplet;
- at least one DNA sugar nucleoside in the Central Base Triplet opposite to the adenosine to be deaminated;
- stabilization of pyrimidine bases outside the Central Base Triplet by 2'-F or 2'-OMe modifications of the nucleoside ribose moieties in roughly equal stoichiometry, whereby a pattern is preferred that avoids blocks of 2'-OMe moieties;
- stabilization of both termini by blocks of three nucleotides which have a double modification, namely a 2'-OMe modification on the sugar moieties and a phosphorothioate linkage.

The chemically modified nucleic acid molecules (oligonucleotides) of the present invention have the advantage that the molecule is sufficiently stable in a vertebrate organism so that the desired effect can be achieved. The molecules according to the present invention are stable against a degradation of different RNases for a sufficient period of time so that an effect can be seen.

Another advantage of the chemically modified nucleic acids of the present invention is that they can be brought directly to the target cells without specific vectors or other helping mechanisms like specific transfection methods. The chemically modified nucleic acids according to the present invention can act via gymnosis, meaning they can be applied directly to the target cells without helping means like vectors or other carriers.

Another advantage of the chemically modified nucleic acids according to the present invention is that they have a high efficiency of editing in clinically relevant targets. The modified nucleic acids can be introduced via gymnosis into the target cells and a comparatively high effect on the translational level in the target cells can be achieved.

Another advantage of the chemically modified nucleic acids according to the present invention is that an editing from A to I can be effected not only with comparatively easily editable targets like 5'UAG but also with more difficult triplets like 5'CAA.

The present invention and preferred embodiments thereof are illustrated by the Examples and the Figures which are, however, not limiting.

The Figures illustrate in particular preferred embodiments of the invention.

Figure 1 shows the basic structure and terminology of two preferred embodiments of the present invention. Figure 1A) shows an asymmetric modified oligonucleotide and Figure 1B) shows a symmetric design.
- *Fig. 1A*: *is an asymmetric embodiment of the current invention comprising of ≥ 4 nucleotides 3'- and ≥ 19 nucleotides 5'-adjacent of the Central Base Triplet (NNN), binding to the targeted RNA. The targeted adenosine (A*) is opposite the central nucleotide of the Central Base Triplet.*
- *Fig. 1B*: *is a symmetric embodiment of the current invention comprising of at least 11 nucleotides 5'-and 3'-adjacent to the Central Base Triplet, binding to the target RNA. The targeted adenosine is opposite the central nucleotide of the Central Base Triplet.*

Figure 2 shows the result of the experiments as performed in Example 1. The degree of editing in the GAPDH open reading frame (ORF) is shown in percent for different oligonucleotides whereby the sequences thereof are provided in the tables. The experiments were performed in the presence (+IFN) and absence of interferon-a.

Figure 3 shows the lead oligonucleotides V120.2 and V117.19 and the editing of GAPDH whereby the experiments are described in more detail in Example 2. Two modified oligonucleotides are shown schematically in Figure 3A) (asymmetrical) and in Figure 3B) (symmetrical). The editing was tested on different cell lines such as HeLa, U2OS, SH-SY5Y, SK-N-BE, Huh7, HepG2, A549, THP-1. The experiments were performed in the presence (+IFN) and absence of interferon-a.

Figure 4 shows the introduction of further chemical modification into preferred GADPH constructs. The more detailed description and details for Figure 4A) and B) can be found in Example 3. Figure 4C) demonstrates the stability and Figure 4D) shows the editing efficacy as disclosed in Example 4.

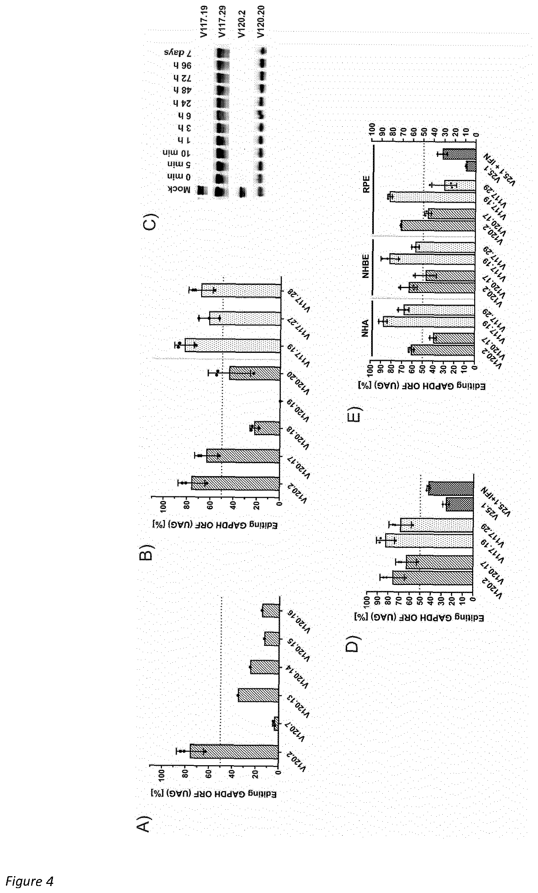

Further details of the graph shown in Figure 4D) and E) can be found in Example 5.

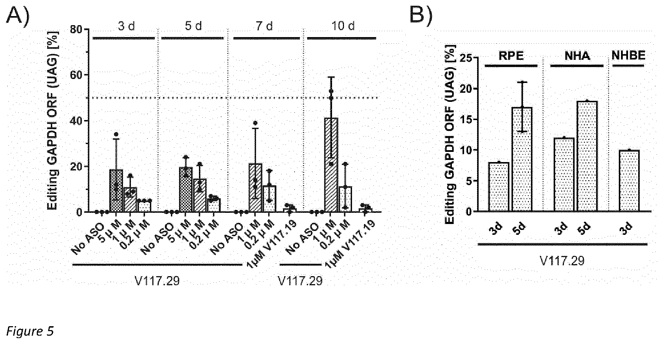

Figure 5 shows the gymnosis with stable chemically modified GADPH-targeting constructs in HeLa cells. The experimental outline is described in more detail in Example 6.

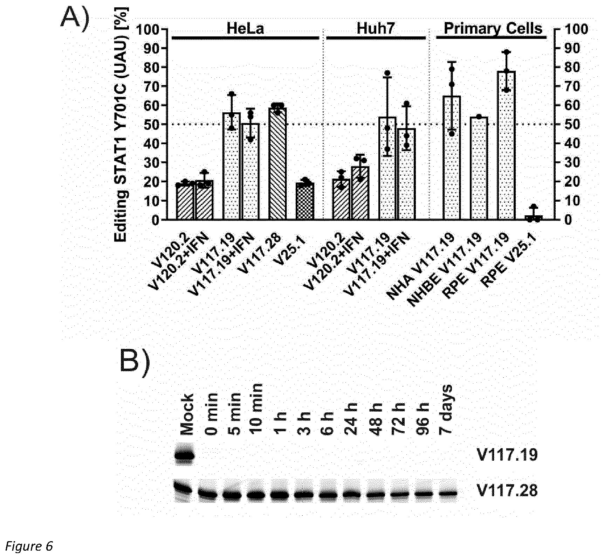

Figure 6 shows the results obtained in Example 7 (Figure 6A)) and in Example 8 for Figure 6B). The data relate to STAT1 Y701 editing and the stability data.

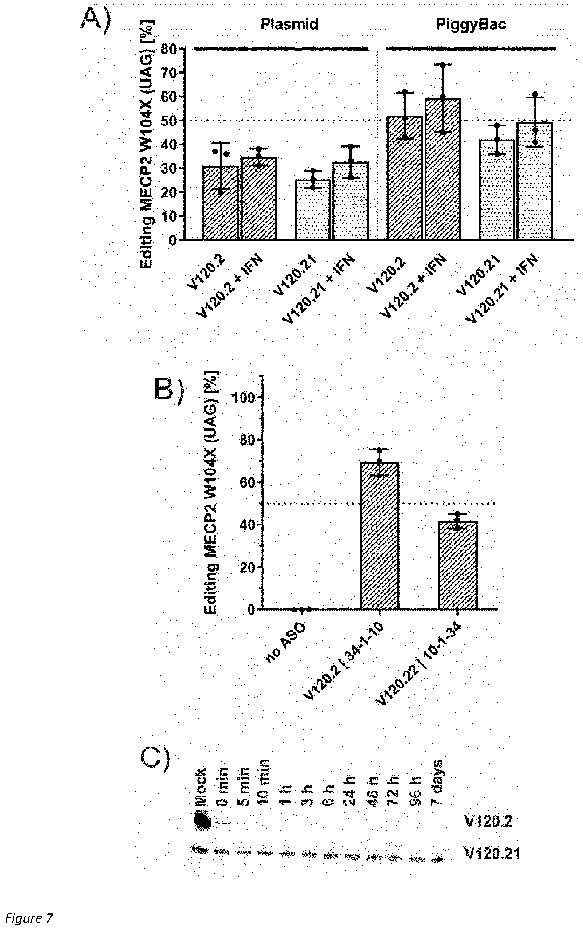

Figure 7 relates to MeCP2 W104X editing and stability data. The details of the results shown in Figure 7A) can be found in Example 9, for Figure 7B) a more detailed description can be found in Example 10 and for Figure 7C) a more detailed description can be found in Example 11.

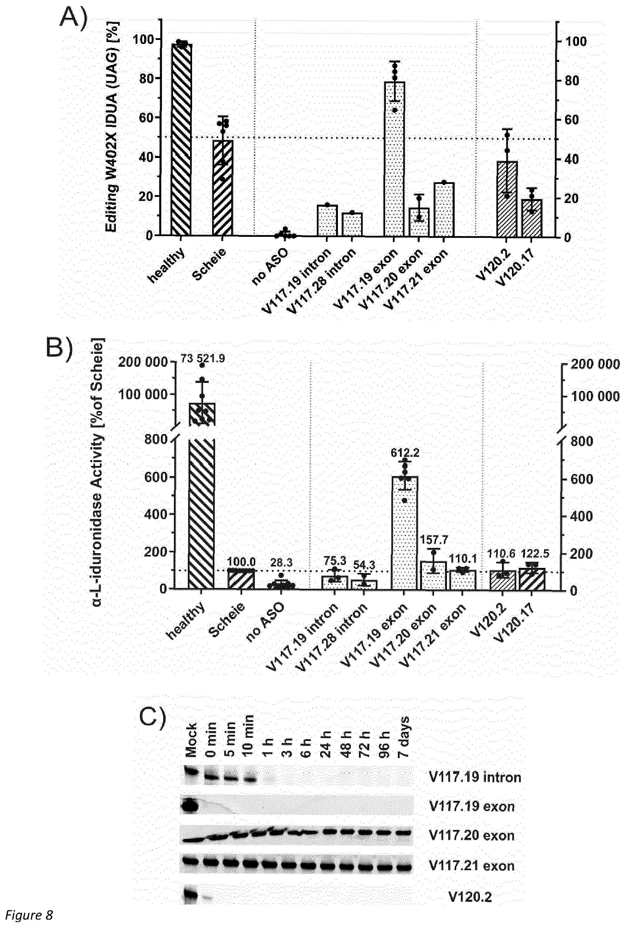

Figure 8 shows hIDUA W402X editing, an α-L-iduronidase assay and stability data for the experimental details. Reference is made to Example 12 for 8A), to Example 13 for 8B) and to Example 14 for 8C).

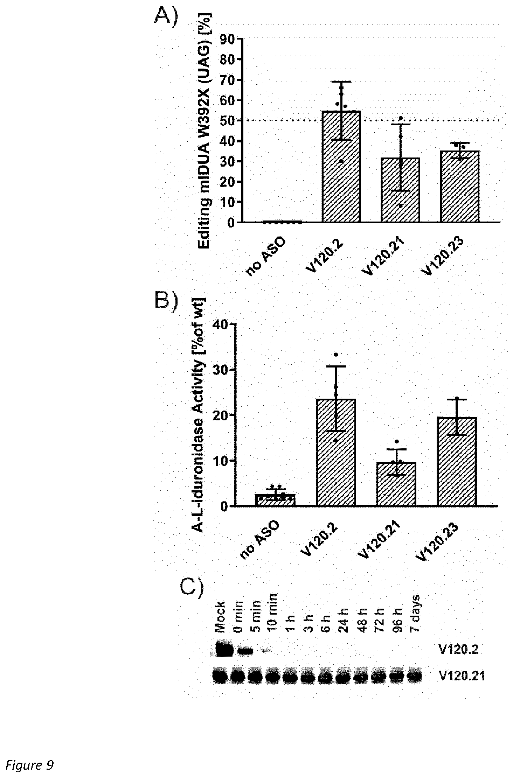

Figure 9 relates to mIDUA W392X editing and the stability data. Figure 9A) shows the results of Example 15, Figure 9B) shows the results of Example 16 and Figure 9C) shows the results of Example 17.

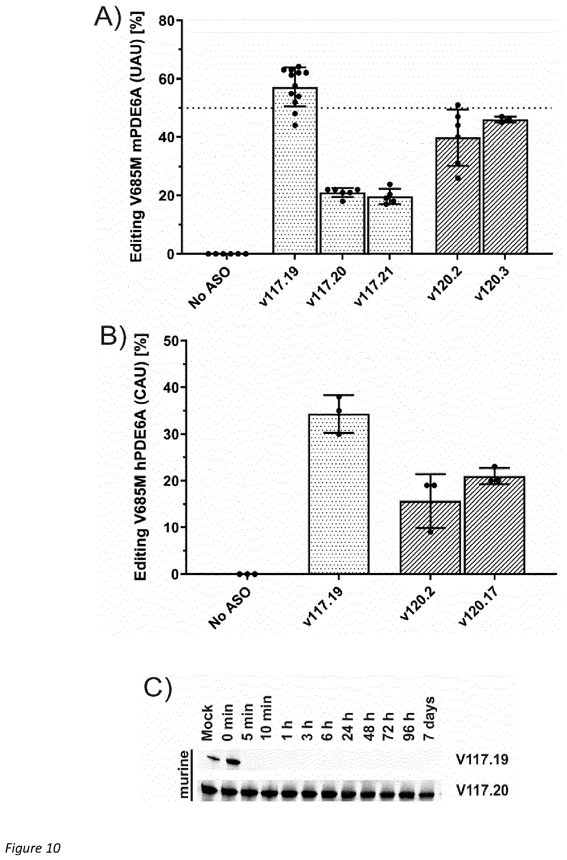

Figure 10 relates to PDE6A V685M editing and the corresponding stability data. The results of Example 18 are shown in Figure 10A), the results of Example 19 are shown in Figure 10B) and the results of Example 20 are shown in Figure 10C).

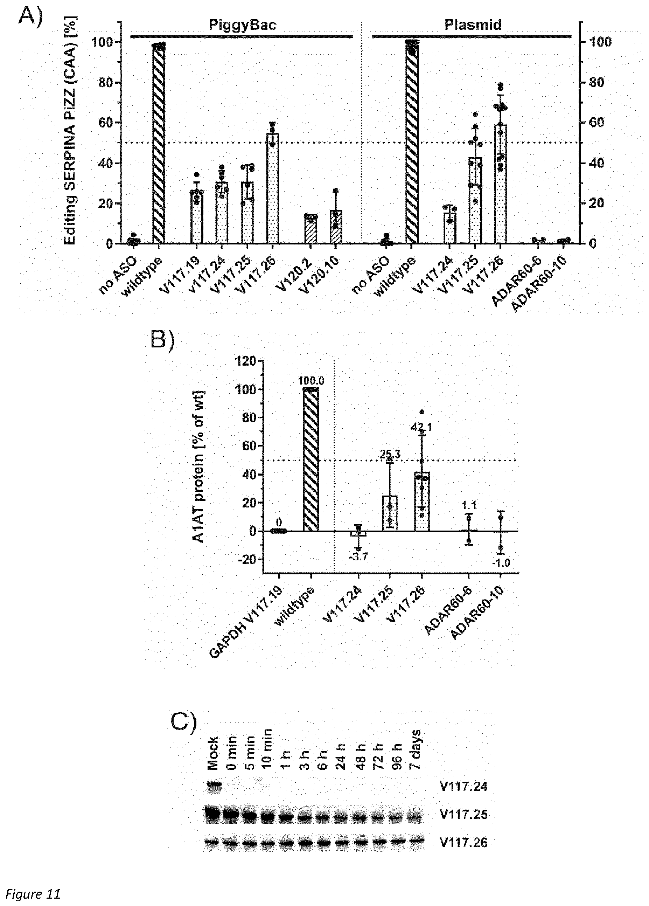

Figure 11 shows the results concerning SERPINA1 PiZZ editing and A1AT ELISA and stability data. For the details of Figure 11A) we refer to Example 21, for Figure 11B) to Example 22 and for Figure 11C) to Example 23.

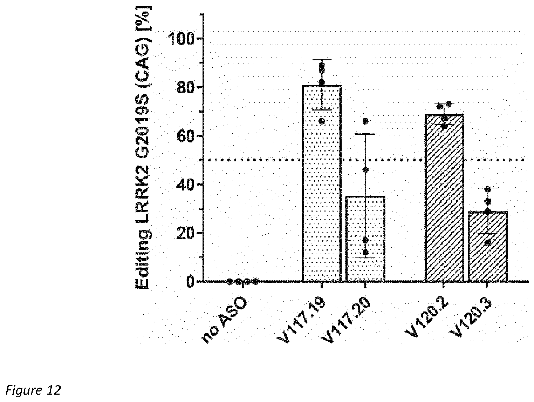

Figure 12 demonstrates the results obtained in Example 24 for the LRRK2 G2019S target. Surprisingly oligonucleotides with the modification according to the present invention (V117.19 and V120.2) show a very high editing yield. By introducing the chemically modified pyrimidine nucleotides according to the teaching of the prior art (V117.20 and V120.3) editing activity of the constructs decrease by about half.

### Example 1: Optimization of RNA editing activity of the constructs on the human housekeeping GAPDH gene at the L157 site (see Figure 2).

The results of Example 1 are shown in Figure 2. It has been shown that long blocks of phosphorothioate (PS) linkages ranging from 2x5 up to 2x25 linkages result in improved editing. Figure 2B) shows that without phosphorothioate linkages no editing can be achieved (construct V117.1). Figures 2C) and 2D) demonstrate the influence of the length of the oligonucleotide. In case an asymmetric design is chosen, the length can be reduced up to 40 nucleotides. Furthermore, it is interesting to note that the addition of interferon-a does not result in an improvement of the editing efficacy.

The effect of the introduction of phosphorothioate linkages in preferred embodiments of the current invention, as well as the most preferred construct lengths and Central Base Triplet positioning was studied. Modified oligonucleotides according to the present invention were used to target an endogenous housekeeping transcript (GAPDH) for editing an adenosine in a 5'UAG context in the open reading frame (ORF) by recruiting endogenous ADARs. Generally, the addition of interferon, depicted as "IFN", to the experimental setting had no significant influence on the editing activity of the constructs. The detailed construct sequences are listed in Table 1.

To obtain the results, HeLa cells (Cat.No.: ATCC CCL-2) were cultured in DMEM plus 10% FBS plus P/S (100U/mL penicillin and 100µg/mL streptomycin. 5×10⁴ cells in 100 µL DMEM plus 10% FBS (+ 600 units IFN-α, Merck, catalog number IF007, lot number 2937858) were added to a transfection mix of 0.5 µL Lipofectamine 2000 and 5 pmol construct/well in a 96-well format. After 24 hours cells were harvested for RNA isolation and sequencing. The following conclusions were drawn from the experiments:
A) Editing activity of the constructs improves with increasing amounts of phosphorothioate (PS) linkages introduced. Construct V117.16 (Seq. ID 2) with only five phosphothioate linkages on each side of the Central Base Triplet shows markedly reduced editing activity compared to construct V117.19 (Seq. ID 5), with 25 PS linkages at both sides.
B) Constructs that do not contain phosphorothioate linkages at all do not show detectable editing in contrast to embodiments that additionally contain PS linkages (V117.19, Seq. ID 5 and V119.4, Seq. ID 8), which are arranged in continuous blocks. In preferred embodiments the oligonucleotide comprises at least one nucleotide block of consecutive phosphorothioate linkages between the nucleotides. The lack of phosphorothioate linkages in the constructs (V117.1, Seq. ID 1 and V119.1, Seq. ID 7) is devastating to editing activity.
C) The editing activities of preferred embodiments of the current invention depend on the length of the oligoribonucleotide. Shortening the oligoribonucleotide arms flanking the Central Base Triplet is detrimental to the editing activity for the symmetric embodiments, e.g. from a total length of 59 nt down to 47 nt (V117.19, Seq. ID 5 and V118.3, Seq. ID 6). For the asymmetric embodiments (V119.4, Seq. ID 8; V120.2, Seq. ID 9; V121.1, Seq. ID 10 and V122.1, Seq. ID 11), a shorter overall oligoribonucleotide is yet sufficient for high editing efficacy compared to the symmetric embodiment, e.g. shorting down to 40 nt (V121.1, Seq. ID 10) is possible, while shorting to 35 nt eliminates editing activity (V122.1, Seq. ID 11).
D) The preferred position of the Central Base Triplet in constructs with the same oligoribonucleotide lengths is important. Longer constructs, e.g. with 59 nucleotides in the current example (V117.19, Seq. ID 5 and V119.4, Seq. ID 8) do not show a clearly preferred position of the Central Base Triplet. Shorter constructs, e.g. with 47 nucleotides (V118.3, Seq. ID 6) or 45 nucleotides length (V120.2, Seq. ID 9), however, show a strong preference to position the Central Base Triplet towards the 3'-end of the construct as for V120.2 (Seq. ID 9), resulting in an asymmetric embodiment of the current invention with a long nucleotide sequence 5'-adjacent to the Central Base Triplet.

**Table 1: Construct sequences and modifications used in Example 1.**

| mN = 2'-O-methyl, N = 2'OH (ribo), * = phosphorothioate linkage | | | |
|---|---|---|---|
| Construct Name | Construct Sequence (5' to 3' direction) | Seq. ID | Figure No. |
| V117.1 | mUmUmG UCAUGGAUGACCUUGGCCAGGGGUG CCA AGCAGUUGGUGGUGCAGGAGGCAUU mGmCmU | 1 | 3B |
| V117.16 | mU*mU*mG* U*C*A UGGAU GA CCUUG GCC AGGGGUG CCA AGCAGUUGGUGGUGCAGGAGGCA*U*U* mG*mC*mU | 2 | 3A |
| V117.17 | mU*mU*mG* U*C*A* U*G*G*A*U GA CCUUG GCC AGGGGUG CCA AGCAGUUGGUGGUGCAGG*A*G*G*C*A*U*U* mG*mC*mU | 3 | 3A |
| V117.18 | mU*mU*mG* U*C*A* U*G*G*A*U* G*A *C*C*U*U*G *G*C*C AGGGGUG CCA   AGCAGUUG*G*U*G*G*U*G*C*A*G*G*A*G*G*C*A*U*U* mG*mC*mU | 4 | 3A |
| V117.19 | mU*mU*mG* U*C*A* U*G*G*A*U*G*A *C*C*U*U*G *G*C*C* A*G*G*G*GUG   CCA AGC*A*G*U*U*G*G*U*G*G*U*G*C*A*G*G*A*G*G*C*A*U*U* mG*mC*mU | 5 | 3A, 3B, 3C, 3D |
| V118.3 | mU*mG*mG* A*U*G*A*C*C*U*U*G*G*C*C*A* G*G*G*GUG   CCA AGC*A*G*U*U* G*G*U*G*G*U*G*C*A*G*G*A* mG*mG*mC | 6 | 3C, 3D |
| V119.1 | mUmCmC UUCCACGAUACCAAAGUUGUCAUGGAUGACCUUGGCCAGGGGUG CCA  AGCAGU mUmGmG | 7 | 3B |
| V119.4 | mU*mC*mC* U*U*C*C*A*C*G*A*U*A*C*C*A*A*A*G*U*U*G*U*C*A*U*G*G*A*U*G*A*C* C*U*U*G*G*C*C*A*G*G*G*GUG CCA AGC*A*G*U* mU*mG*mG | 8 | 3B, 3C, 3D |
| V120.2 | mC*mA*mA* A*G*U*U*G*U*C*A*U*G*G*A*U*G*A*C*C*U*U*G*G*C*C*A* G*G*G*GUG CCA AGC*A*G*U* mU*mG*mG | 9 | 3C, 3D |
| V121.1 | mU*mU*mG* U*C*A*U*G*G*A*U*G*A*C*C*U*U*G*G*C*C*A* G*G*G*GUG CCA AGC*A*G*U* mU*mG*mG | 10 | 3C |
| V122.1 | mA*mU*mG* G*A*U*G*A*C*C*U*U*G*G*C*C*A* G*G*G*GUG CCA AGC*A*G*U* mU*mG*mG | 11 | 3C |

### Example 2: Editing of the human GAPDH transcript at the L157 site with endogenous ADAR in different tumor cell lines.

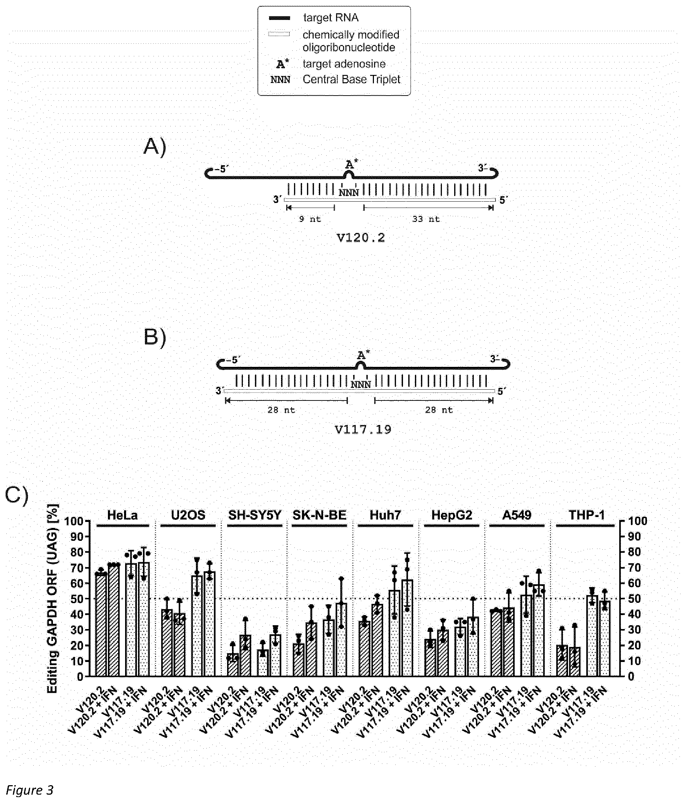

In order to investigate the applicability of the constructs of the current invention in cell lines derived from different tissues, a cell line screen was performed with two particularly preferred embodiments of the current invention: long and symmetric design (V117.19, Seq. ID 5, Figure 3B) and short and asymmetric design (V120.2, Seq. ID 9, Figure 3A). A list of the constructs showing the modifications is provided in Table 2.

Different tumor cell lines were cultured in DMEM plus 10% FBS plus P/S, except for THP-1 which were cultured in RPMI plus 10% FBS. 1×10⁵ cells/well (for HeLa cells (cat. no. ATCC CCL-2), U2OS-Flp-In T-REx32 (kind donation from Elmar Schiebel), Huh7 (CLS GmbH, Heidelberg, cat. no. 300156), HepG2 (DSMZ, Braunschweig, Germany, cat. no. ACC180) and A549 (European Collection of Authenticated Cell Cultures ECACC 86012804)) were seeded in a 24-well plate. For SK-NBE(2) (cat. no. ATCC CRL-2271) and SH-SY5Y (cat. no. ATCC CRL-2266) cell lines, 2.5x 10⁵ cells per 24-well were seeded. After 24 h medium was changed (supplemented with 3,000 U/well IFN-α for IFN-treated cells) and cells were forward transfected with a transfection mix of 25 pmol ASO/well in 50µL OptiMEM and 1.5 µl/well Lipofectamine RNAiMAX Reagent (ThermoFisher Scientific) in 50µL OptiMEM. Both solutions were combined after 5 min incubation and incubated for an additional 20 min before the transfection mix was distributed evenly into one well. After 24 h cells were harvested for RNA isolation and sequencing.

THP-1 were transfected the same way after 3x 10⁵ cells/well of a 24-well plate were differentiated for 3 days in RPMI plus 10% FBS plus PMA (200nM) and cultured for 5 days in RPMI plus 10% FBS afterwards.

As shown in Figure 3C, both constructs showed editing activity in all cell lines tested. Notably, the editing activity could not be severely improved through the addition of interferon ("+ IFN"), as was the case for a prior art (disclosed in WO 2020/001793).

**Table 2: Construct sequences and modifications as used in Example 2**

| mN = 2'-O-methyl, N = 2'OH (ribo), * = phosphorothioate linkage | | | |
|---|---|---|---|
| Construct Name | Construct Sequence (5' to 3' direction) | Seq. ID | Figure No. |
| V117.19 | mU*mU*mG* U*C*A* U*G*G*A*U*G*A *C*C*U*U*G *G*C*C* A*G*G*G*GUG   CCA AGC*A*G*U*U*G*G*U*G*G*U*G*C*A*G*G*A*G*G*C*A*U*U* mG*mC*mU | 5 | 3B |
| V120.2 | mC*mA*mA* A*G*U*U*G*U*C*A*U*G*G*A*U*G*A*C*C*U*U*G*G*C*C*A* G*G*G*GUG CCA AGC*A*G*U* mU*mG*mG | 9 | 3A |

### Example 3: Introduction of chemical modification patterns into constructs targeting the L157 site of the endogenous human GAPDH transcript with endogenous ADAR.

The results of this example are shown in Figure 4A) and B). The use of large 2'-O-methyl blocks as used for example in construct V120.7 (Seq. ID 13) leads to a substantial decrease of editing (Figure 4A), Figure 4B) shows that concerning the modification of the pyrimidine nucleosides, 2'F modification is well accepted, a 2'-O-methyl modification is more or less accepted, but 2'-MOE is not accepted.

Modifications prove essential for synthetic oligonucleotides to stay intact in a therapeutic setting, where there are multiple lines of defense against non-self oligonucleotides. These modifications can be, but are not limited to, the sugar moiety of the nucleosides, such as the 2'-O-methyl, 2'-fluoro (2'F), 2'-MOE or 2'-deoxy (2'H) group instead of the 2'-hydroxy group (2'OH). Besides the different types of modifications, the position of modified nucleosides in the constructs is also an important aspect. Both the type of modifications, as well as the position of modified nucleosides have a strong effect on the editing activity of the constructs. A list of the sequences and their modifications tested is provided in Table 3.

HeLa cells were cultured and forward transfected as described in Example 2. In brief, 1×₁₀⁵ cells per 24-well were seeded and forward transfected with 25 pmol of the respective construct 24 h later. After 24 h post-transfection, the cells were harvested for RNA isolation and Sanger sequencing.

As shown in Figure 4A, the introduction of long blocks of 2'-O-methyl modifications (V120.7, Seq. ID 13), a hallmark of prior art (e.g. EP 3 507 366)), has a devastating effect on editing activity. However, also other repetitive patterns of modifications, e.g. 2'F/2'F/ribo (V120.13, Seq. ID 14), 2'-O-methyl/2'F (V120.14, Seq. ID 15), 2'-O-methyl/2'F/2'F (V120.15, Seq. ID 16), or 2'-deoxy/2'F/2'F (V120.16, Seq. ID 17) had a rather strong impact on editing efficiency. In contrast, chemical modification of a number of selected nucleotides in the constructs was well accepted (Figure 4B). In these embodiments, the pyrimidine bases inside the Central Base Triplet were modified with 2'desoxy and combined with other modifications at all pyrimidine nucleosides outside the Central Base Triplet. In particular, the 2'-F modification was very well accepted (V120.17, Seq. ID 18 and V117.28, Seq. ID 23), whereas the 2'-O-methyl modification alone reduced editing yield depending on the design of the construct (V120.18, Seq. ID 19 and V117.27, Seq. ID 22). The well-known modification 2'-MOE was not accepted when placed on all pyrimidines (V120.19, Seq. ID 20). Overall, the data show that the nature of the chemical modification as well as the sites of their placement within the oligoribonucleotide have a distinct effect on the RNA editing yield.

**Table 3: Construct sequences and modifications from Example 3**

| mN = 2'-O-methyl, fN = 2'-fluoro, N = 2'OH (ribo), dN = 2'H (deoxyribo), <N> = 2'-MOE, * = phosphorothioate linkage | | | |
|---|---|---|---|
| Construct Name | Construct Sequence (5' to 3' direction) | Seq. ID | Figure No. |
| V120.2 | mC*mA*mA* A*G*U*U*G*U*C*A*U*G*G*A*U*G*A*C*C*U*U*G*G*C*C*A* G*G*G*GUG CCA AGC*A*G*U* mU*mG*mG | 9 | 4A, 4B |
| V120.7 | mC*mA*mA*mA*mG*mU*mU*mG*mU*mC*mA*mU*mG*mG*mA*mU* mG*mA*mC*mC*mU*mU*mG*mG*mC*mC*mA* mG*mG*mG*mGmUmG CCA mAmGmC*mA*mG*mU*mU*mG*mG | 13 | 4A |
| V120.13 | mC*mA*mA* A* fG*fU* U* fG* fU* C* fA*fU* G* fG*fA* U* fG*fA*C* fC*fU* U* fG*fG* C* fC*fA* G*G*G*GUG  CCA  AG C* A*G* U* mU*mG*mG | 14 | 4A |
| V120.14 | mC*fA*mA* fA* mG*fU* mU* fG* mU* fC* mA* fU* mG* fG*mA* fU*mG*fA*mC*fC*mU*fU*mG*fG*mC*fC*mA*G*G*G*GUG CCA AG C* A*G* U*mU*mG*mG | 15 | 4A |
| V120.15 | mC*fA*fA* mA* fG*fU* mU* fG* fU* mC* fA*fU* mG*fG*fA* mU* fG*fA*mC*fC*fU*mU*fG*fG*mC*fC*fA* G*G*G*GUG CCA AG C* A*G* U*mU*mG*mG | 16 | 4A |
| V120.16 | dC*fA*fA* dA* fG*fU* dT* fG* fU* dC* fA*fU* dG* fG*fA* dT* fG*fA*dC*fC*fU*dT*fG*fG*dC*fC*fA* G*G*G*GUG CCA AG C* A*G* U* mU*mG*mG | 17 | 4A |
| V120.17 | mC*mA*mA* A*G* fU* fU* G* fU* fC* A* fU* G*G*A* fU* G*A* fC* fC*fU* fU* G*G* fC*fC* A* G*G*G*G fU G  dCdC A  AG fC* A*G* fU* mU*mG*mG | 18 | 4B |
| V120.18 | C6-Disulfide linker  mC*mA*mA*A*G*mU* mU*G*mU* mC*A*mU*G*G*A*mU*G*A*mC* mC*mU*mU*G*G*mC*mC*A GG*G*G mUG   dCdC A  AG mC*A*G*mU*mU*mG* mG | 19 | 4B |
| V120.19 | C6-Disulfide linker <C*A*A*> A*G* <U* U*> G* <U* C*> A* <U*> G*G*A* <U*> G*A* <C* C*U* U*> G*G* <C*C*> A GG*G*G <U> G dCdC A AG <C*> A*G* <U*U*G* G> | 20 | 4B |
| V120.20 | C6-Disulfide linker mC*mA*mA*A*G* fU* fU* G* fU* fC* A* fU* G*G*A* fU* G*A* fC* fC*fU* fU* G*G* fC*fC* A GG*G*G fU G  dCdC A  AG fC* A*G* fU* mU*mG*mG | 21 | 4B |
| V117.19 | mU*mU*mG* U*C*A* U*G*G*A*U*G*A *C*C*U*U*G *G*C*C* A*G*G*G*GUG   CCA AGC*A*G*U*U*G*G*U*G*G*U*G*C*A*G*G*A*G*G*C*A*U*U* mG*mC*mU | 5 | 4B |
| V117.27 | C6-Disulfide linker  mU*mU*mG*mU*mC* A*mU*G*G*A*mU*G*A *mC*mC*mU*mU*G *G*mC*mC*A*G*G*G*GmUG  dCdC A AGmC*A*G* mU*mU*G*G*mU*G*G*mU*G*mC*A*G*G*A*G*G*mC*A*mU*mU*mG* mC*mU | 22 | 4B |
| V117.28 | C6-Disulfide linker  mU*mU*mG* fU*fC*A* fU* G*G*A* fU*G*A *fC*fC*fU*fU* G *G* fC*fC* A*G*G*G*G fU G   dCdC A   AG fC* A*G* fU*fU* G*G* fU* G*G* fU* G* fC* A*G*G*A*G*G* fC* A* fU*fU* mG*mC*mU | 23 | 4B |

### Example 4: Stabilization of constructs targeting the endogenous L157 GAPDH gene with endogenous ADAR.

Figure 4C) and Figure 4D) show the effect of the modification concerning the stability against nucleases in 10% FBS (Figure 4C)). Figure 4D) shows that the improved oligonucleotides are sufficiently stable, comparatively short and nevertheless have a high efficacy.

In order to evaluate the therapeutic potential of the current invention, the determination of the stability of the constructs from the current invention against the endogenous defense mechanisms against non-self oligonucleotides, such as RNases, is of great importance. The introduction of chemically modified nucleosides into the construct can improve stability against RNases. To determine this, four preferred embodiments of the current invention - two with chemical modifications (V117.29, Seq. ID 24 and V120.20, Seq. ID 21) and two without chemical modifications (V117.19, Seq. ID 5 and V120.2, Seq. ID 9) at the 2'-hydroxyl group of the sugar moiety - were tested for their stability in fetal bovine serum over the course of 7 days. The sequences and modifications are found in Table 4.

The constructs were incubated in 10% fetal bovine serum (FBS). The degradation of the oligonucleotides was visualized by denaturing urea PAGE. In the experiments, 15 pmol of the respective ASO were diluted in 10 µl PBS plus 10% FBS. Mock samples contained 15 pmol ASO diluted in PBS only. All samples were incubated at 37°C for the given time points, then frozen with liquid N₂ and stored immediately at -80°C. Denaturation of the samples was achieved by adding 7 µl RNA loading dye (1:10 dilution of Rotiphorese^{®} Sequencing gel buffer concentrate in Rotiphorese^{®} Sequencing gel diluent, Carl Roth) each and incubation at 70°C for 2 min. Denatured samples were then loaded on a urea (7 M) polyacrylamide (15%) electrophoresis (PAGE) gel and run for 4-6 h at 1200 V in 1 x TBE buffer. Bands were visualized through a SYBR^{™} Gold Nucleic Acid Gel Stain (Thermo Fisher Scientific) according to manufacturer's instructions and scanned at the excitation wavelength λₑₓ = 473 nm with a Fujifilm FLA-5100 Fluorescent Image Analyzer.

Irrespective of the design of the construct, e.g. the length of the oligoribonucleotide or the position of the Central Base Triplet, the two constructs lacking the chemical modifications at the 2'-hydroxyl group of the nucleoside sugar moiety (i.e. V117.19, Seq. ID 5 and V120.2, Seq. ID 9) were immediately degraded. In contrast, the two constructs bearing chemical modifications at the 2'-hydroxyl group of the pyrimidine nucleosides (V117.29, Seq. ID 24 and V120.20, Seq. ID 21) were able to withstand 10% fetal bovine serum for more than 7 days. Thus, the inclusion of chemical modifications on the sugar moiety of the constructs at selected nucleosides increases durability against serum RNase degradation and thus longevity of the constructs in serum.

Furthermore, we benchmarked the chemically stabilized embodiments (V120.17, Seq. ID 18 and V117.29, Seq. ID 24) side-by-side with an oligoribonucleotide design from the prior art (V25.1, Seq. ID 12, disclosed in WO 2020/001793) for the editing of the endogenous GAPDH transcript by recruiting endogenous HeLa ADAR (Figure 4D). Specifically, we found that both chemically stabilized embodiments clearly outcompete the prior art oligonucleotide V25.1 (Seq. ID 12) by means of editing efficiency. This was also true when the editing efficiency with the prior art oligoribonucleotide was boosted by IFN-α treatment ("+ IFN").

**Table 4: Construct sequences and modifications used in Example 4**

| mN = 2'-O-methyl, fN = 2'-fluoro, N = 2'OH (ribo), **dN** = 2'H (deoxyribo), {N} = LNA, * = phosphorothioate linkage | | | |
|---|---|---|---|
| Construct Name | Construct Sequence (5' to 3' direction) | Seq. ID | Figure No. |
| V117.19 | mU*mU*mG* U*C*A* U*G*G*A*U*G*A *C*C*U*U*G *G*C*C* A*G*G*G*GUG   CCA AGC*A*G*U*U*G*G*U*G*G*U*G*C*A*G*G*A*G*G*C*A*U*U* mG*mC*mU | 5 | 4C, 4D |
| V117.29 | C6-Amino linker - mU*mU*mG* fU*fC* A* fU* G*G*A* fU*G*A* fC*fC*fU*fU* G*G* fC*fC* A*G*G*G*G fU G  dCdC A  AG fC* A*G* fU*fU* G*G* fU* G*G* fU* G* fC* A*G*G*A*G*G* fC* A* fU*fU* mG*mC*mU | 24 | 4C, 4D |
| V120.2 | mC*mA*mA* A*G*U*U*G*U*C*A*U*G*G*A*U*G*A*C*C*U*U*G*G*C*C*A* G*G*G*GUG CCA AGC*A*G*U* mU*mG*mG | 9 | 4C, 4D |
| V120.20 | C6-Disulfide linker - mC*mA*mA*A*G* fU* fU* G* fU* fC* A* fU* G*G*A* fU* G*A* fC* fC*fU* fU* G*G* fC*fC* A GG*G*G fU G   dCdC A  AG fC* A*G* fU* mU*mG*mG | 21 | 4C, 4D |
| V25.1 (WO 2020/001793) | mG*mG*mU G mUmC GAGAAGAGGAGAA mC AA mU A mU G mCmU AAA mU G mUmU G mUmUmCmUmC G mUmCmUmCmCmUmC GA mC A mCmC UUGUCAUGGAUGACCUUGGCCA mG {G} mGmGmUmG CCA mAmGmCmA {G*} mU*mU* {G*} mG - C6-Amino linker | 12 | 4D |

### Example 5: Editing of the human GAPDH transcript at the L157 site with endogenous ADAR in different cell types (see Figure 4D + E).

Figure 4E) shows the results of Example 5 whereby the experiments were performed in human primary cells. These experiments are very important since they show that the invention works in cell lines which have a direct link to the later commercial use.

In order to investigate the applicability of the constructs of the current invention in HeLa cells (Figure 4D) and primary cells (Figure 4E) from different tissues, a cell screen was performed with a total of four particularly preferred embodiments of the current invention, either without chemically modified pyrimidine nucleosides (V117.19, Seq. ID 5 and V120.2, Seq. ID 9) or with chemically modified pyrimidine nucleosides (V117.29, Seq. ID 24 and V120.17, Seq. ID 18). A list of the constructs used and their corresponding sequences is in the table below (Table 5).

Normal human astrocytes (NHA, Lonza cat. no. CC-2565) were cultured in ABM Basal Medium (Lonza cat. no. CC-3187) with AGM SingleQuot Kit Supplementary & Growth Factors (Lonza cat. no. CC-4123), human retinal pigment epithelial cells (H-RPE, Lonza cat. no. 00194987) were cultured in RtEBM Basal Medium (Lonza cat. no. 00195406) supplemented with RtEGM Retinal Epithelial Cell Growth Medium SingleQuots Supplements and Growth Factors (Lonza ca. no. 00195407) without FBS (for seeding FBS was added and after 24 h medium was changed to FBS-free medium), and normal human bronchial epithelial cells (NHBE, Lonza cat. no. CC-2540) were cultured in BEGM Bronchial Epithelial Cell Growth Basal Medium (Lonza cat. no CC-3171) supplemented with BEGM Bronchial Epithelial Cell Growth Medium SingleQuots Supplements and Growth Factors (CC-4175). The transfection procedure was performed with 1 × 10⁵ cells/well seeded and 25 pmol ASO transfected, as described in Example 3 for HeLa cells. IFN-treated cells ("+ IFN") were supplemented with 15,000 U/well IFN-α before the transfection of the construct. Cells were harvested for RNA isolation and Sanger sequencing after 24 h.

HeLa cells were treated and forward transfected as already described in Example 3 with 1 × 10⁵ cells/well seeded and 25 pmol ASO transfected, and corresponding wells treated with IFN as described for the primary cells.

As shown in Figure 4D and E, both constructs showed efficient editing activity in all cell types (HeLa, NHA, NHBE and RPE cells) tested. The inclusion of additional chemical modifications at the pyrimidine nucleosides was well accepted in most cases. However, the constructs bearing chemically modified nucleosides (V117.29, Seq. ID 24 and V120.17, Seq. ID 18) showed a reduction of editing activity compared to their counterpart constructs without chemically modified nucleosides (V117.19, Seq. ID 5 and V120.2, Seq. ID 9). Notably, the editing activity of all embodiments of the invention was strongly improved compared to a prior art disclosed in WO 2020/001793 (V25.1, Seq. ID 12). This was also true after boosting the editing with V25.1 oligoribonucleotide with the addition of interferon-a ("+ IFN").

**Table 5: Construct sequences and modifications used in Example 5**

| mN = 2'-O-methyl, fN = 2'-fluoro, N = 2'OH (ribo), **dN** = 2'H (deoxyribo), * = phosphorothioate linkage, {N} = LNA | | | |
|---|---|---|---|
| Construct Name | Construct Sequence (5' to 3' direction) | Seq. ID | Figure No. |
| V117.19 | mU*mU*mG* U*C*A* U*G*G*A*U*G*A *C*C*U*U*G *G*C*C* A*G*G*G*GUG   CCA AGC*A*G*U*U*G*G*U*G*G*U*G*C*A*G*G*A*G*G*C*A*U*U* mG*mC*mU | 5 | 4D, 4E |
| V117.29 | C6-Amino linker - mU*mU*mG* fU*fC* A* fU* G*G*A* fU*G*A* fC*fC*fU*fU* G*G* fC*fC* A*G*G*G*G fU G  dCdC A  AG fC* A*G* fU*fU* G*G* fU* G*G* fU* G* fC* A*G*G*A*G*G* fC* A* fU*fU* mG*mC*mU | 24 | 4D, 4E |
| V120.2 | mC*mA*mA* A*G*U*U*G*U*C*A*U*G*G*A*U*G*A*C*C*U*U*G*G*C*C*A* G*G*G*GUG CCA AGC*A*G*U* mU*mG*mG | 9 | 4D, 4E |
| V120.17 | mC*mA*mA* A*G* fU* fU* G* fU* fC* A* fU* G*G*A* fU* G*A* fC* fC*fU* fU* G*G* fC*fC* A* G*G*G*G fU G  dCdC A  AG fC* A*G* fU* mU*mG*mG | 18 | 4D, 4E |
| V25.1 (WO 2020/001793) | mG*mG*mU G mUmC GAGAAGAGGAGAA mC AA mU A mU G mCmU AAA mU G mUmU G mUmUmCmUmC G mUmCmUmCmCmUmC GA mC A mCmC UUGUCAUGGAUGACCUUGGCCA mG {G} mGmGmUmG CCA mAmGmCmA {G*} mU*mU* {G*} mG - C6-Amino linker | 12 | 4D, 4E |

### Example 6: Gymnotic uptake of constructs to edit the endogenous human GAPDH transcript at the L157 site with endogenous ADAR.

The results of this example are shown in Figure 5. It has been shown that the constructs according to the invention allow a so-called "naked uptake" which means that the oligonucleotides reach their target site in cancer cell lines and human primary cells without auxiliary measures. It should be noted that without a modification at the pyrimidine nucleosides the naked uptake is less effective.

Preferred embodiments of the present invention were also tested for their ability to edit a human housekeeping gene (GAPDH) without the aid of transfection agents (gymnosis). The constructs used for this example are listed in Table 6.

HeLa cells were cultivated in DMEM plus 3 % FBS. For this, 10⁴ HeLa cells per 24-well were seeded in 500 µl medium/well and incubated for 24 h. After 24 h, the medium was changed to medium containing 5 µM, 1 µM, 0.2 µM and 0 µM ASO. Each well contained 300 µl construct-containing medium and was changed after 5 days. HeLa cells were additionally detached and split by 50 % after 5 days. Cells were harvested for RNA isolation and Sanger sequencing after 3 days, 5 days, 7 days and 10 days.

For the primary cells, 10⁴ cells/well RPE, NBE and NHA were seeded into 24-well plates in the respective medium and after 24 h the medium was replaced with 250 µL medium and 50µL ASO in OptiMEM was added with a final ASO concentration of 5 µM. Cells were harvested for RNA isolation after 3 or 5 days after addition of the oligonucleotide.

As shown in Figure 5A, construct v117.29 (Seq. ID 24) is readily taken up into the treated HeLa cells and shows high editing activity without the addition of transfection agents. Best results were achieved when HeLa cells were treated with 1 µM construct for 10 days. The editing effect was dose-dependent as shown by variation of the concentration of V117.29 (5 µM or 0.2 µM) in the medium. Notably, the addition of 1 µM construct V117.19 (Seq. ID 5), which lacks the additional stabilizing chemical modifications compared to V117.29 and which is quickly degraded in serum (see Example 4), shows almost no editing activity in HeLa cells after treatment for 7 and 10 days, although V117.29 performs best under these conditions. This clearly shows that the chemical modification pattern as disclosed in this invention is capable and required to achieve editing under gymnotic uptake of the oligonucleotide. The treated primary cells (RPE, NHA and NHBE cells, shown in Figure 5B) also show that sufficient amounts of the construct V117.29 are taken up without the aid of transfection reagents to achieve editing. Much like the 1 µM condition in HeLa cells, the editing effect increases after 5 days compared to 3 days of treatment. Conclusively, gymnotic uptake of the stabilized constructs is possible in both cell lines and primary cells, and is efficient enough to achieve editing of endogenous transcript by harnessing endogenous ADAR.

**Table 6: Construct sequences and modifications used in Example 6**

| mN = 2'-O-methyl, fN = 2'-fluoro, N = 2'OH (ribo), **dN** = 2'H (deoxyribo), * = phosphorothioate linkage | | | |
|---|---|---|---|
| Construct Name | Construct Sequence (5' to 3' direction) | Seq. ID | Figure No. |
| V117.19 | mU*mU*mG* U*C*A* U*G*G*A*U*G*A *C*C*U*U*G *G*C*C* A*G*G*G*GUG   CCA AGC*A*G*U*U*G*G*U*G*G*U*G*C*A*G*G*A*G*G*C*A*U*U* mG*mC*mU | 5 | 5A |
| V117.29 | C6-Amino linker  mU*mU*mG* fU*fC* A* fU* G*G*A* fU*G*A* fC*fC*fU*fU* G*G* fC*fC* A*G*G*G*G fU G   dCdC A   AG fC* A*G* fU*fU* G*G* fU* G*G* fU* G* fC* A*G*G*A*G*G* fC* A* fU*fU* mG*mC*mU | 24 | 5A, 5B |

### Example 7: Editing of endogenous human STAT1 transcript at the Y701 site with endogenous ADAR.

The results of Example 7 are shown in Figure 6. A clear stabilization can be recognized and a strong improvement of the editing yield compared with prior art (v25.1). In primary cells the editing yield is improved by the factor of about 10. It can be concluded that the chemical stabilization of the oligonucleotides is well accepted whereby a high editing yield is obtained.

As previously done for constructs targeting the endogenous human GAPDH L157 site in Example 2, three preferred constructs of the current invention targeting the endogenous human STAT1 transcript at the phosphorylation site Y701 were tested in two different cell lines (Huh7 and HeLa cells), and three different primary cells (NHA, NHBE and RPE cells). The constructs were both versions, chemically stabilized (V117.28, Seq. ID 27) and not chemically stabilized (V120.2, Seq. ID 25 and V117.19, Seq. ID 26). The sequences of the constructs used for this example are listed in Table 7.

Tumor cell lines were treated as described in Example 2, while primary cells were treated as described in Example 5. In brief, 1x10⁵ cells per 24-well were forward transfected with 25 pmol construct and the RNA isolated 24 h later for Sanger sequencing.

As shown in Figure 6A, V117.19 showed high editing activity in all cell types tested. The inclusion of additional stabilizing chemical modifications at the pyrimidine nucleosides in the construct V117.28 did not compromise the editing activity compared to its corresponding less stable construct, V117.19 (see Figure 6B). The editing activity of both symmetric constructs was improved nearly 3-fold compared to the prior art V25.1 (Seq. ID 28) in HeLa cells, and more than 8-fold improved in RPE cells, even though V117.19 is shorter and more chemically stabilized. The asymmetric embodiment, V120.2, also showed editing activity, however, lower compared to both V117.19 and V117.28. For none of the new constructs, the editing activity could be boosted through the addition of interferon-a ("+ IFN"), as was the case for the prior art V25.1 (disclosed in WO 2020/001793). Overall, the invention gave clearly better editing yields, independent of IFN treatment, and with a shorter than the prior art solution.

**Table 7: Construct sequences and modifications used in Example 7 and Example 8**

| mN = 2'-O-methyl, fN = 2'-fluoro, N = 2'OH (ribo), **dN** = 2'H (deoxyribo), * = phosphorothioate linkage, {N} = LNA | | | |
|---|---|---|---|
| Construct Name | Construct Sequence (5' to 3' direction) | Seq. ID | Figure No. |
| V120.2 | mA*mA*mC* U*U*C*A*G*A*C*A*C*A*G*A*A*A*U*C*A*A*C*U*C*A*G*U*C*U*U *GAU ACA UCC*A*G*U*mU*mC*mC | 25 | 5A |
| V117.19 | mC*mA*mG*A*C*A*C*A*G*A*A*A*U*C*A*A*C*U*C*A*G*U*C*U* U*GAU  ACA UCC*A*G*U*U*C*C*U*U*U*A*G*G*C*C*A*U*C*A*A*G*U* mU*mC*mC | 26 | 5A, 5B |
| V117.28 | mC*mA*mG*A* fC*A* fC*A*G*A*A*A* fU*fC* A*A* fC*fU*fC*A*G* fU*fC*fU*fU*GA fU dAdCdA fU fC fC*A*G* fU*fU*fC*fC*fU*fU*fU* A*G*G*G* fC*fC*A* fU*fC* A*A*G* fU* mU*mC*mC | 27 | 5A, 5B |
| V25.1 (WO 2020/001793) | mG*mG*mUGmUmCGAGAAGAGGAGAAmCAAmUAmUGmCmU A AAmUGmUmUGmUmUmCmUmCGmUmCmUmCmCmUmCGAC ACCCA GACACAGAAAUCAACUCAGUmC{T}mUmGmAmU ACA  mUmCmCmA{G*}mU*mU*{C*}mC - C6-Amino linker | 28 | 5A |

### Example 8: Stabilization of constructs targeting the endogenous Y701 site of the human STAT1 gene with endogenous ADAR (see Figure 6B).

As previously discussed in Example 4, the stabilization of constructs against defensive mechanisms in endogenous settings such as RNases is essential to enable a therapeutic perspective on the invention. For this, a preferred embodiment of the current invention with (V117.28, Seq. ID 27) and without (V117.19, Seq. ID 26) was tested for its stability in 10% fetal bovine serum over the course of 7 days. The sequences and modifications are found in Table 7. Results were obtained as described already in Example 4.

As shown in Figure 6B, the inclusion of the modified pyrimidine nucleosides into the construct (V117.28) strongly improves the longevity in 10% FBS compared to the construct lacking the chemically modified nucleosides (V117.19). Together with the superior editing activity of the constructs in Example 7 compared to V25.1 (Seq. ID 28, disclosed in WO 2020/001793), these constructs are a clear improvement over the prior art.

### Example 9: Editing of the disease-causing W104X mutation of the murine MECP2 transcript with endogenous ADAR.

Figure 7 shows a high editing efficacy in a disease-relevant model system. Figure 7 shows a good yield also after stabilization as in particular shown in the FBS assay (Figure 7C)). Figure 7B) demonstrates that also in asymmetric embodiments there is a preferred asymmetric structure as shown by comparison of V120.2 versus V120.22.

Constructs targeting the disease-causing W104X mutation of the murine MECP2 gene were tested via two different approaches: in HeLa cells transfected with the target cDNA on plasmid or that have the disease-causing MECP2 W104X mutated cDNA stably integrated into their genome via the piggyBac transposase system. The construct was either unstable (V120.2, Seq. ID 29) or additionally chemically stabilized with modifications (V120.21, Seq. ID 30). The sequences of the constructs used for this example are listed in Table 8.

For the plasmid-transfected approach ("Plasmid"), 5 x 10⁴ HeLa cells were seeded in a 24-well plate. After 24 h cells were forward transfected with a plasmid containing the murine MECP2 W104X cDNA gene. 300 ng plasmid and 0.9 µl FuGENE^{®} 6 (Promega) were each diluted in 50 µl Opti-MEM and incubated for 5 min, then combined and incubated for an additional 20 min. The medium was changed (supplemented with 15,000 U/well IFN-α for IFN-treated cells), and the transfection mix evenly distributed into one well. 24 h after plasmid transfection, cells were forward transfected with 25 pmol construct/well and 1.5 µl/well Lipofectamine RNAiMAX Reagent (ThermoFisher Scientific). After 24 h, cells were harvested for RNA isolation and sequencing.

For the piggyBac approach ("PiggyBac"), 1 x 10⁵ HeLa cells containing the murine MECP2 W104X cDNA gene stably integrated into their genome via the piggyBac transposase system were seeded in a 24-well plate. After 24 h cells were forward transfected as described for the plasmid approach with 25 pmol construct/well and 1.5 µl/well Lipofectamine RNAiMAX Reagent (ThermoFisher Scientific). IFN-treated cells were supplemented with 3,000 U/well IFN-α before the transfection of the construct. After 24 h, cells were harvested for RNA isolation and sequencing.

As shown in Figure 7A, efficient editing activity to comparable level was possible with both, V120.2, the unstable construct, and V120.21, the construct stabilized with chemically modified pyrimidine nucleosides. While both constructs performed equally well in the plasmid-borne approach, a small reduction of the V120.21 compared to V120.2 could be seen in the piggyBac approach. As already seen in previous examples also, neither of the constructs show a strong difference in editing activities after the addition of interferon ("+ IFN").

**Table 8: Construct sequences and modifications used in Example 10**

| mN = 2'-O-methyl, fN = 2'-fluoro, N = 2'OH (ribo), **dN** = 2'H (deoxyribo), * = phosphorothioate linkage | | | |
|---|---|---|---|
| Construct Name | Construct Sequence (5' to 3' direction) | Seq. ID | Figure No. |
| V120.2 | mU*mC*mG*G*C*C*A*G*A*C*U*U*C*C*U*U*G*U*U*A*A*G*C* U*U*U*C*G*UGU   CCA ACC*U*U*C*mA*mG*mG | 29 | 7A, 7B, 7C |
| V120.21 | mU*mC*mG*G* fC*fC* A*G*A* fC*fU*fU*fC*fC*fU*fU*fU* G* fU*fU*fU*A*A*G* fC*fU*fU*fU*fC*G*fU G fU   dCdC A A fC fC*fU*fU*fC*  mA*mG*mG | 30 | 7A, 7C |
| V120.22 | mC*mU*mU*  U*C*G*UGU CCA ACC*U*U*C*A*G*G*C*A*A*G*G*U*G*G*G*U*C*A*U*C*A*U*A*C* A*U*mA*mG*mG | 31 | 7B |

### Example 10: Effect of inversion of the asymmetric general construct design on editing yields of the disease-causing W104X mutation of the murine MECP2 transcript with endogenous ADAR (see Figure 7B).

For the asymmetric construct it was already shown that positioning the Central Base Triplet towards the 3'-end of short construct improves editing activity compared to symmetric constructs with the same length. The basic design of the asymmetric construct, in particular for V120.2 (Seq. ID 29) in Example 3A was inverted, so that the Central Base Triplet-which is usually placed at the 3'-end of the construct - is found at the 5'-end of the construct (V120.22, Seq. ID 31). The sequences of the constructs used for this example are listed in Table 8. HeLa cells were treated as previously described for the plasmid-transfected approach in Example 9.

As shown in Figure 7B, inverting the usual position of the Central Base Triplet (V120.2, Seq. ID 29) to the opposite site (V120.22, Seq. ID 31) has a detrimental effect on the editing activity, reducing it almost by half. Thus, it is clear that the positioning of the Central Base Triplet for asymmetric embodiments is preferred towards the 3'-end of the oligonucleotide.

### Example 11: Stabilization of constructs targeting the disease-causing W104X mutation of the murine MECP2 transcript with endogenous ADAR (see Figure 7C).

As previously discussed in Example 4, the stabilization of constructs against defensive mechanisms in endogenous settings such as RNases is essential to enable a therapeutic perspective on the invention. For this, a preferred embodiment of the current invention with (V120.21, Seq. ID 30) and without (V120.2, Seq. ID 29) chemically modified pyrimidine nucleosides was tested for its stability in 10 % fetal bovine serum over the course of 7 days. The sequences and modifications are found in Table 8. Results were obtained as described previously in Example 4.

As shown in Figure 7C, the inclusion of the modified pyrimidine nucleosides into the construct (V120.21) allows the construct to withstand in 10% FBS for over 7 days, while the unstable construct (V120.2) is almost immediately degraded.

### Example 12: Editing of the disease-causing W402X mutation in the human IDUA transcript in Hurler syndrome patient fibroblasts with endogenous ADAR (Figure 8A).

Figure 8 shows the results in a disease-related test system. The target RNA is endogenously expressed in fibroblasts of patients. Although the stabilization is achieved, it is disadvantageous with regard to the editing efficacy.

Germline mutations in the IDUA gene are a frequent cause for mucopolysaccharidosis type I, a lysosomal storage disease where the encoded enzyme of the IDUA gene, α-L-iduronidase, is defective and unable to participate in the degradation of polysaccharides. This leads to the accumulation of glycosaminoglycans (GAGs) in the lysosomes and shows varying degrees of severity: ranging from the milder Scheie syndrome, where patients have minor health issues and a normal lifespan, to the more severe Hurler syndrome, where patients suffer from major health issues, up to lethality during childhood. The homozygous W402X mutation in the IDUA gene is one of the underlying causes of the severe Hurler syndrome, and is the desired target of the corresponding constructs in this example in attempt to edit the diseased RNA and restore the enzyme function (see Example 13). Corresponding construct sequences can be found in Table 9.

Fibroblasts from patients with the milder Scheie syndrome ("Scheie", GM01323), the severe Hurler syndrome (GM06214) and from a healthy donor ("healthy", GM05659) were purchased from the Coriell Institute for Medical Research (USA). Fibroblasts were cultivated in DMEM containing 15 % FBS. 2.5 x 10⁵ cells/well in 2.5 ml DMEM plus 15 % FBS were seeded into 6-well plates. Forward transfection was performed 24 h after seeding with 125 pmol construct and 7.5 µl Lipofectamine RNAiMAX Reagent, each diluted in 250 µl Opti-MEM. Both solutions were combined after 5 min incubation and incubated for an additional 20 min before the transfection mix was distributed evenly into one well. Scheie syndrome and healthy fibroblasts were treated with transfection mix without addition of any construct. The medium was changed 24 h after transfection. 48 h after transfection, fibroblasts were harvested for RNA isolation and sequencing.

As shown in Figure 8A, symmetric embodiments targeting the intronic sequence surrounding the target adenosine (V117.19 intron, Seq. ID 35 and V117.28 intron, Seq ID 36) showed only low editing activity. In contrast, the construct targeting the mature mRNA sequence around the target adenosine (V117.19 exon, Seq. ID 32) shows very high editing activity, surpassing the corrected mRNA ratios compared to those found in the Scheie syndrome fibroblasts. However, upon addition of stabilizing modifications into the construct (V117.20 exon, Seq. ID 33 and V117.21 exon, Seq. ID 34), the editing yields suffered compared to their less stable counterpart, v117.19 exon. Finally, the asymmetric embodiment V120.2 (Seq. ID 37) also shows good editing activity, reaching levels close to those found in Scheie syndrome fibroblasts. For the asymmetric embodiment, the addition of stabilizing modifications at pyrimidine nucleosides into the construct (V120.17, Seq.ID 38) also reduces the editing efficiency to some extent.

**Table 9: Construct sequences and modifications used in Example 12, Example 13 and Example 14**

| mN = 2'-O-methyl, fN = 2'-fluoro, N = 2'OH (ribo), **dN** = 2'H (deoxyribo), * = phosphorothioate linkage | | | |
|---|---|---|---|
| Construct Name | Construct Sequence (5' to 3' direction) | Seq. ID | Figure No. |
| V117.19 exon | mG*mG*mA*C*G*G*U*C*C*C*G*G*C*C*U*G*C*G*A*C*A*C*U*U*C*GGC CCA GAG*C*U*G*C*U*C*C*U*C*A*U*C*C*A*G*C*A*G*C*G*C*C*mA*mG*mC | 32 | 8A, 8B, 8C |
| V117.20 exon | mG*mG*mA* fC*G*G*fU*fC*fC*fC*G*G*fC*fC*fU*G*fC*G*A*fC*A*fC*fU*fU*fC*GGfC dCdCdA GAG*fC*fU*G*fC*fU*fC*fC*fU*fC*A*fU*fC*fC*A*G*fC*A*G*fC*G*fC*fC* mA*mG*mC | 33 | 8A, 8B, 8C |
| V117.21 exon | mG*mG*mA* fC*G*G*mU* fC* mC* fC*G*G* mC* fC* mU*G* fC*G*A* mC*A*fC* mU* fU* mC*GG fC  dCdCdA  GAG* mC*fU*G*mC* fU* mC* fC* mU* fC*A* mU* fC* mC* A*G* fC*A*G* mC*G* fC* mC*mA*mG*mC | 34 | 8A, 8B, 8C |
| V117.19 intron | mG*mG*mA*C*G*G*U*C*C*C*G*G*C*C*U*G*C*G*A*C*A*C*U*U*C*GGC CCA  GAG*C*U*G*C*U*C*C*U*C*A*U*C*U*G*C*G*G*G*C*G*G* mG*mG*mG | 35 | 8A, 8B, 8C |
| V117.28 intron | mG*mG*mA* fC* G*G* fU*fC*fC*fC* G*G* fC*fC*fU* G* fC* G*A* fC* A* fC*fU*fU*fC* GG fC  dCdC A  GAG* fC*fU* G* fC*fU*fC*fC*fU*fC* A* fU*fC*fU* G* fC* G*G*G*G* fC* G*G* mG*mG*mG | 36 | 8A, 8B |
| V120.2 | mG*mU*mC*C*A*G*G*A*C*G*G*U*C*C*C*G*G*C*C*U*G*C*G*A*C*A*C* U*U*C*GGC CCA GAG*C*U*G* mC*mU*mC | 37 | 8A, 8B, 8C |
| V120.17 | mG*mU*mC* fC* A*G*G*A* fC* G*G* fU*fC*fC*fC* G*G* fC*fC*fU* G* fC* G*A* fC* A* fC*fU*fU*fC* GG fC  dCdC A  GAG* fC*fU* G* mC*mU*mC | 38 | 8A, 8B |
| ADAR65-30 (WO 2018/041973) | mC*mU*mG*mU*mCmCmAmAmCmAmCmAmGmCmCmCmCmAmGmC mCmUmUmUmGmAmGmAmCmCmUmCmUmGmU dC*dC*dA* mGmAmGmUmUmGmUmU*mC*mU*mC*mC | 39 | 8A, 8B |

### Example 13: α-L-iduronidase assay of cell lysate from Hurler syndrome patient fibroblasts treated with constructs targeting the disease-causing W402X mutation in the human IDUA transcript (Figure 8B).

In attempt to restore the protein function of W402X mutated α-L-iduronidase through RNA editing with the constructs (described in Example 12), an α-L-iduronidase assay was performed with the lysate of fibroblasts treated with different constructs. Corresponding construct sequences can be found in Table 9.

Patient fibroblasts were treated, seeded and transfected as described in Example 12. For each tested construct, two 6-wells were used. 48 h after transfection, fibroblasts were detached and washed once with PBS. 40 µl 0.5 % Triton X-100 in PBS were added to the cell pellet and incubated on ice for 30 min and the α-L-iduronidase enzyme assay was performed. A standard dilution series of 4-methylumbelliferone was prepared (0 - 160 µM). For each concentration of the standard dilution series, 25 µl of the standard solution were added to 25 µl 0.4 M sodium formate buffer (pH 3.5) in a 96-well plate. For the fibroblast samples, 25 µl of each solution were added to a 96-well and mixed with 25 µl substrate solution (180 µM 4-methylumbelliferyl α-L-iduronide in 0.4M sodium formate buffer, pH 3.5). The reaction was incubated at 37°C for 90 min and the enzyme activity stopped by adding 200 µl glycine carbonate butter (pH 10.4) to the well. The fluorescence of 4-methylumbelliferone was measured with an excitation wavelength of λₑₓ = 355nm at an emission wavelength of λₑₘ = 460nm with a Tecan Spark 10M plate reader. Calculated enzyme activities were referenced to the protein amount (determined by BCA assay) and standardized to the enzyme activity of Scheie lysate.

As seen in Figure 8B, results were standardized to the enzyme activity of α-L-iduronidase in the lysate of Scheie syndrome fibroblasts (set to 100 %), which also determines the lowest necessary therapeutic threshold for protein restoration. As a reference, untreated Hurler syndrome fibroblasts ("no ASO") showed approximately 3-fold lower α-L-iduronidase activity, while the healthy fibroblasts ("healthy") showed about 700-fold higher α-L-iduronidase activity of that observed in Scheie syndrome fibroblasts.

Both constructs targeting the intronic IDUA mRNA (V117.19 intron, Seq. ID 35 and V117.28 intron, Seq. ID 36), similarly to their corresponding editing activities, did not achieve very efficient protein restoration, reaching α-L-iduronidase activity levels between half and three-quarters the amount as in Scheie syndrome fibroblasts.

The asymmetric constructs (V120.2, Seq. ID 37 and V120.17, Seq. ID 38), as well as the stabilized symmetric constructs (V117.20 exon, Seq. ID 33 and V117.21 exon, Seq. ID 34) all show α-L-iduronidase activity levels similar to, or slightly above those found in Scheie syndrome fibroblasts. Finally, the unstable symmetric construct targeting the mature mRNA, V117.19 exon (Seq. ID 32), shows α-L-iduronidase activity levels 6-fold over Scheie syndrome fibroblasts levels. Overall, this indicates that the invention is capable of restoring protein function to a level that promises a therapeutic effect. This was also true for some embodiments carrying the full stabilizing chemical modification pattern even though this affectedediting efficiency.

### Example 14: Stabilization of constructs targeting the disease-causing W402X mutation of the human IDUA transcript with endogenous ADAR (see Figure 8C).

As discussed in Example 4, the stabilization of constructs against defensive mechanisms in endogenous settings such as RNases is essential to enable a therapeutic perspective on the invention. For this, preferred embodiments of the current invention with (V120.17, Seq. ID 38; V117.20 exon, Seq. ID 33; V117.21 exon, Seq. ID 34 and V117.28 intron, Seq. ID 36) and without (V120.2, Seq. ID 37; V117.19 intron, Seq. ID 35 and V117.19 exon, Seq. ID 32) stabilizations by introduction of chemical modifications at pyrimidine nucleosides were tested for their stability in 10 % fetal bovine serum over the course of 7 days. The sequences and modifications are found in Table 9. Results were obtained as described previously in Example 4.

As shown in Figure 8C, the inclusion of the modified pyrimidine nucleosides into the construct (V120.17, Seq. ID 38; V117.20 exon, Seq. ID 33; V117.21 exon, Seq. ID 34 and V117.28 intron, Seq. ID 36) allows the construct to withstand in 10% FBS for over 7 days, while the unstable constructs (V120.2, Seq. ID 37; V117.19 intron, Seq. ID 35 and V117.19 exon, Seq. ID 32) are almost immediately degraded. Overall, this clearly demonstrates the stabilizing effect of the introduced chemical modifications.

### Example 15: Editing of the disease-causing W392X mutation in the murine IDUA transcript with endogenous ADAR.

Figure 9 shows the results of Example 15 (Figure 9A)), for Example 16 (Figure 9B)) and for Example 17 (Figure 9C)).

The W392X mutation in the murine IDUA transcript is equivalent to the W402X mutation in the human IDUA gene, which is described briefly in Example 12. Constructs targeting the murine disease-causing mutant were tested for their editing activity and protein restoration (see Example 16). Construct sequences are depicted in Table 10.

5x10⁴ HeLa cells were seeded in a 24-well plate. After 24 h cells were forward transfected with a plasmid containing the murine W392X mutated IDUA cDNA. 300 ng plasmid and 0.9 µl FuGENE^{®} 6 (Promega) were each diluted in 50 µl Opti-MEM and incubated for 5 min, then combined and incubated for an additional 20 min. The medium was changed, and the transfection mix evenly distributed into one well. 24 h after plasmid transfection, cells were forward transfected with 25 pmol ASO/well and 1.5 µl/well Lipofectamine RNAiMAX Reagent (ThermoFisher Scientific). After 24 h, cells were harvested for RNA isolation and sequencing.

As shown in Figure 9A, relatively good editing activity is achieved with the unstable construct, V120.2 (Seq. ID 40). Both constructs bearing stabilizing modifications (V120.21, Seq. ID 41 and V120.23, Seq. ID 42) loose some part of their editing activities compared to V120.2. Construct V120.23, which bears an alternating mixture of 2'-fluoro and 2'O-methyl modifications at the sugar moieties of the pyrimidine nucleosides, shows a slightly higher editing activity compared to V120.21, which bears only 2'-fluoro modifications, showing that mixing 2'-F with 2'O-methyl can support editing efficiency.

**Table 10: Construct sequences from Example 15, Example 16 and Example 17**

| mN = 2'-O-methyl, fN = 2'-fluoro, N = 2'OH (ribo), **dN** = 2'H (deoxyribo), * = phosphorothioate linkage | | | |
|---|---|---|---|
| Construct Name | Construct Sequence (5' to 3' direction) | Seq. ID | Figure No. |
| V120.2 | mG*mU*mC*C*A*A*C*A*C*A*G*C*C*C*A*G*C*C*U*U*U*G*A*G*A *C*C*U*C*UGC CCA GAG*U*U*G* mU*mU*mC | 40 | 9A, 9B, 9C |
| V120.21 | mG*mU*mC* fC* A*A* fC* A* fC* A*G* fC*fC*fC*fC* A*G* fC* fC* fU* fU* fU* G*A*G*A* fC*fC*fU*fC*fU G fC dCdC A GAG* fU* fU*G* mU*mU*mC | 41 | 9A, 9B, 9C |
| V120.23 | mG*mU*mC*mC*A*A* fC* A*  mC* A*G*  fC* mC* fC*  mC* A*G* fC* mC* fU* mU* fU* G*A*G*A*mC* fC* mU* fC* mU G fC  dCdC A GAG* mU* fU*G*  mU*mU*mC | 42 | 9A, 9B |

### Example 16: α-L-iduronidase assay of cell lysate from HeLa cells treated with constructs targeting the disease-causing W392X mutation in the murine IDUA transcript (Figure 9B).

To determine the transfer of edited IDUA RNA to restored α-L-iduronidase protein, a α-L-iduronidase enzyme assay was performed. Absolute activity results were relativized to α-L-iduronidase activity from HeLa cells transfected with a plasmid containing the murine IDUA wildtype cDNA. Construct sequences are provided in Table 10.

HeLa cells were treated, seeded and transfected as described in Example 15 with 5x10⁴ cells, 300 ng plasmid and 25 pmol construct per 24-well. To provide the wildtype reference data, HeLa cells were transfected with a plasmid containing the murine IDUA wildtype cDNA. For each setting, one 24-well was used. 24 h after the transfection of the construct, cells were lysed in 100 µl M-PER buffer (Thermo Scientific) for the α-L-iduronidase enzyme assay. Further treatment of the samples was performed as described for Example 13, save for the determination of the protein amount, which was achieved via Bradford Assay.

As seen in Figure 9B, the α-L-iduronidase assay results depict what is already evident from the editing data from Figure 9A (Example 15). Both constructs bearing stabilizing modifications (V120.21, Seq. ID 41 and V120.23, Seq. ID 42) have partly lowered α-L-iduronidase activity compared to V120.2 (Seq. ID 40). Compared to the editing results however, construct V120.23 - with an alternating pattern of 2'-fluoro and 2'O-methyl modifications at the pyrimidine nucleosides - shows a clearly higher editing activity compared to V120.21, which only has 2'-fluoro modifications at the pyrimidine nucleosides showing that mixing 2'-F with 2'O-methyl can support editing efficiency.

### Example 17: Stabilization of constructs targeting the disease-causing W392X mutation of the murine IDUA transcript with endogenous ADAR (Figure 9C).

As discussed in Example 4, construct stabilization against RNases, such as those found in serum, is essential to enable a therapeutic perspective on the invention. For this, preferred embodiments of the current invention with (V120.21, Seq. ID 41) and without (V120.2, Seq. ID 40) stabilizations by introduction of chemical modifications at pyrimidine nucleosides were tested for their stability in 10% fetal bovine serum over the course of 7 days. Construct sequences are provided in Table 10. Results were obtained as described in Example 4.

As shown in Figure 9C, the inclusion of the modified pyrimidine nucleosides into the construct (V120.21) allows the construct to withstand in 10% FBS for over 7 days, while the unstable construct (V120.2) are almost immediately degraded, showing again the stabilizing effect obtained.

### Example 18: Editing of the disease-causing V685M mutation in the murine PDE6A transcript with endogenous ADAR (Figure 10A).

The two preferred embodiments V117.19 and V120.2 were previously tested for multiple other targets (scheme depicted in Figure 3A and B). The editing activity of the constructs designed for the disease-causing V685M mutation site in murine PDE6A gene, which causes gradual blindness through *Retinitis pigmentosa* and is caused by an adenosine in a 5'-UAU-3' context, was investigated. A list of the constructs is provided in Table 11.

5x 10⁴ HeLa cells were seeded in a 24-well plate. After 24 h cells were forward transfected with a plasmid containing the disease-causing murine PDE6A V685M cDNA. 300 ng plasmid and 0.9 µl FuGENE^{®} 6 (Promega) were each diluted in 50 µl Opti-MEM and incubated for 5 min, then combined and incubated for an additional 20 min. The medium was changed, and the transfection mix evenly distributed into one well. 24 h after plasmid transfection, cells were forward transfected with 25 pmol ASO/well and 1.5 µl/well Lipofectamine RNAiMAX Reagent (ThermoFisher Scientific). After 24 h, cells were harvested for RNA isolation and sequencing.

As shown in Figure 10A, the unstable, symmetric construct (V117.19, Seq. ID 43) shows the highest editing activity. However, once the construct bears stabilizing modifications (V117.20, Seq. ID 44 and V117.21, Seq. ID 45), editing activity of the constructs drops, similar as seen in Example 12 for the symmetric constructs targeting the W402X site of human IDUA gene (Seq. ID 32-36).

For the asymmetric constructs, the contrary can be observed. The unstable construct V120.2 (Seq. ID 46) has slightly lower editing activity compared to the stabilized construct, V120.3 (Seq. ID 47). The drop in editing activity upon stabilization as for the symmetric construct is not observed.

**Table 11: Construct sequences and modifications used in Example 18 and Example 20**

| mN = 2'-O-methyl, fN = 2'-fluoro, N = 2'OH (ribo), **dN** = 2'H (deoxyribo), * = phosphorothioate linkage | | | |
|---|---|---|---|
| Construct Name | Construct Sequence (5' to 3' direction) | Seq. ID | Figure No. |
| V117.19 | mG*mG*mU*A*C*U*C*U*C*A*U*A*U*G*U*C*U*U*U*G*A*C*U*G*A* UCC ACA AUC*U*U*C*U*G*G*A*A*C*A*U*G*G*U*C*C*U*U*U*C*U* mU*mG*mA | 43 | 10A, 10C |
| V117.20 | C6- Amino linker - mG*mG*mU*A* fC*fU*fC*fU*fC* A* fU* A*fU* G* fU*fC*fU*fU*fU* G*A* fC*fU* G*A* fUfCfC dAdCdA A fUfC*fU*fU*fC*fU*  G*G*A*A* fC* A* fU* G*G* fU*fC*fC*fU*fU*fU*fU*fC*fU* mU*mG*mA | 44 | 10A, 10C |
| V117.21 | C6- Amino linker - mG*mG*mU* A* fC*mU* fC* mU* fC* A* mU*A* fU* G* mU*fC* mU* fU* mU*G*A* fC* mU* G*A* fU mC fC dAdCdA A mU fC* mU* fU* mC* fU* G*G*A*A* mC*A* fU* G*G* mU* fC* mC* fU*mU* fU* mU* fC* mU*mU*mG*mA | 45 | 10A |
| V120.2 | mU*mC*mC* U*G*G*G*U*A*C*U*C*U*C*A*U*A*U*G*U*C*U*U*G*A*C*U*G*A*U CC ACA AUC*U*U*C* mU*mG*mG | 46 | 10A |
| V120.3 | mU*mC*mC* fU*G*G*G*fU*A* fC*fU*fC*fU*fC* A* fU* A* fU*G* fU*fC*fU*fU*fU* G*A* fC*fU*G*A* fUfCfC dAdCdA A fUfC*fU*fU*fC* mU*mG*mG | 47 | 10A |

### Example 19: Editing of the disease-causing V685M mutation in the human PDE6A transcript with endogenous ADAR (Figure 108).

The embodiments V117.19 and V120.2 are strongly preferred for numerous other targets already presented. As described previously for Example 18, the V685M mutation in the PDE6A gene - this time human - is another potential site the current invention can be applied to. In contrast to the murine gene, the target adenosine in the human gene is part of a 5'-CAU-3' context. A list of the constructs is provided in Table 12.

Results were obtained as described for Example 18. However, instead of transfecting the HeLa cells with a plasmid containing the murine PDE6A gene, a plasmid containing the mutated human PDE6A V685M cDNA was transfected.

While is it clear that the overall editing yields are not as high as for the murine context in Example 18, it is evident that the constructs editing activity presented in Figure 10B also proves applicability of the invention to the chosen setting and gene. The overall lower editing activities may be caused by the lesser preferred editing context for ADAR to detect the target adenosine in the human gene (5'-CAU) compared to the more preferred context in the murine gene (5'-UAU).

V117.19 (Seq. ID 48) shows the highest overall editing activity. Interestingly, as already seen for the murine gene in Example 18, the stable asymmetric construct V120.17 (Seq. ID 50) shows slightly higher editing activity than its corresponding unstable construct V120.2 (Seq. ID 49).

**Table 12: Construct sequences and modifications used in Example 19**

| mN = 2'-O-methyl, fN = 2'-fluoro, N = 2'OH (ribo), **dN** = 2'H (deoxyribo), * = phosphorothioate linkage | | | |
|---|---|---|---|
| Construct Name | Construct Sequence (5' to 3' direction) | Seq. ID | Figure No. |
| V117.19 | mU*mU*mC*A*C*U*C*U*C*A*U*A*U*G*U*C*U*U*A*G*A*C*U*G*A*U CC  ACG AUC*U*U*U*U*G*G*A*A*C*A*U*C*G*U*C*C*U*C*U*U*C*U* mU*mG*mA | 48 | 10B |
| V120.2 | mU*mC*mC*U*G*U*U*C*A*C*U*C*U*C*A*U*A*U*G*U*C*U*U*A*G*A *C*U*G*A*UCC ACG AUC*U*U*U*mU*mG*mG | 49 | 10B |
| V120.17 | mU*mC*mC* fU* G* fU*fU*fC* A* fC*fU*fC*fU*fC* A* fU* A* fU* G* fU*fC*fU*fU* A*G*A* fC*fU* G*A* fUfCfC **dAdCdG** A fUfC*fU*fU*fU* mU*mG*mG | 50 | 10B |

### Example 20: Stabilization of constructs targeting the disease-causing V685M mutation of the murine PDE6A transcript with endogenous ADAR (Figure 10C).

As discussed in Example 4, constructs that are stable against endogenous defense mechanisms, such as RNases found in serum, are preferred for the therapeutic perspective of this invention. For this, preferred embodiments used in Example 18 with (V117.20, Seq. ID 44) and without (V117.19, Seq. ID 43) stabilizations by introduction of chemical modifications at pyrimidine nucleosides were tested for their stability in 10% fetal bovine serum over the course of 7 days. Construct sequences are provided in Table 11. Results were obtained as described in Example 4.

As shown in Figure 10C, the inclusion of the modified pyrimidine nucleosides into the construct (V117.20, Seq. ID 44) allows the construct to withstand in 10% FBS for over 7 days, while the unstable construct (V117.19, Seq. ID 43) are almost immediately degraded.

### Example 21: Editing of the disease-causing E342K mutation in the human SERPINA 1 transcript with endogenous ADAR (Figure 11A).

The SERPINA1 gene encodes the α-1-antitrypsin (A1AT) protein, an important protease inhibitor synthesized in the liver that protects particularly thin tissues from self-digestion. From the liver it is eventually secreted into the bloodstream, where it is spread systemically. Germline mutations in the SERPINA1 gene can cause impaired functioning of A1AT of varying severity. The severe E342K mutation causes misfolding of the A1AT protein, whereby the A1AT protein is no longer properly secreted into the bloodstream. This causes liver disease due to excessive A1AT accumulation and lung disease due to lack of A1AT. The target adenosine underlying the E342K mutation is in a 5'-CAA-3' context.

The constructs targeting the disease-causing E342K mutation of the human SERPINA1 gene were tested via two different approaches: in HeLa cells transfected with the target cDNA on plasmid or that have the disease-causing E342K SERPINA1 mutated cDNA stably integrated into their genome via the piggyBac transposase system. The constructs were either unstable (V117.19, Seq. ID 51 and V120.2, Seq. ID 55) or chemically stabilized with modified pyrimidine nucleosides (V117.24, Seq. ID 52; V117.25, Seq. ID 53, V117.26, Seq. ID 54 and V120.10, Seq. ID 56). The sequences of the constructs used for this example are listed in Table 13.

For the plasmid-transfected approach ("Plasmid"), 2.5x10⁴ HeLa cells were seeded in a 24-well plate. After 24 h cells were forward transfected with a plasmid containing the human SERPINA1 E342K mutated cDNA or the SERPINA1 healthy cDNA ("wildtype"). 300 ng plasmid and 0.9 µl FuGENE^{®} 6 (Promega) were each diluted in 50 µl Opti-MEM and incubated for 5 min, then combined and incubated for an additional 20 min. The medium was changed, and the transfection mix evenly distributed into one well. 24 h after plasmid transfection, cells were forward transfected with 5 pmol construct/well and 1.5 µl/well Lipofectamine RNAiMAX Reagent (ThermoFisher Scientific). After 24 h, medium was changed. 48 h after transfection, cells were harvested for RNA isolation and sequencing.

For the piggyBac transposase approach ("PiggyBac"), 1 x 10⁵ HeLa cells containing the human SERPINA1 E342K mutated cDNA gene or SERPINA1 wildtype gene ("wildtype") stably integrated into their genome via the piggyBac transposase system were seeded in a 24-well plate. After 24 h cells were forward transfected with 25 pmol construct/well and 1.5 µl/well Lipofectamine RNAiMAX Reagent (ThermoFisher Scientific). After 24 h, cells were harvested for RNA isolation and sequencing.

As shown in Figure 11A, the editing activities of the unstable, symmetric constructs V117.19 (Seq. ID 51) and V117.24 (Seq. ID 52) as well as the stable and unstable asymmetric constructs V120.2 (Seq. ID 55) and V120.10 (Seq. ID 56) are moderate in both the piggyBac and plasmid approach. However, upon introduction of an inosine base at the furthermost 3'-site of the Central Base Triplet instead of a guanosine base, the editing activities of the constructs (V117.25, Seq. ID 53 and especially V117.26, Seq. ID 54) shows a strong increase, although both constructs bear stabilizing modifications, which has usually caused a reduction in editing activity compared to the corresponding unstable construct. Again, as already seen in Example 16 (Fig. 9), the mixture of 2'-F and 2'-O-methyl instead of only 2'-F at the pyrimidine nucleosides (V117.25 vs. 117.26) improved editing yields.

**Table 13: Construct sequences and modifications from Example 21, Example 22 and Example 23**

| mN = 2'-O-methyl, fN = 2'-fluoro, N = 2'OH (ribo), **dN** = 2'H (deoxyribo), * = phosphorothioate linkage | | | |
|---|---|---|---|
| Construct Name | Construct Sequence (5' to 3' direction) | Seq. ID | Figure No. |
| V117.19 | mC*mA*mU*G*G*C*C*C*A*G*C*A*G*C*U*U*C*A*G*U*C*C*C*U* UUC UCG UCG*A*U*G*G*U*C*A*G*C*A*C*A*G*C*C*U*U*A*U*G*C*A*mC*mG *mG | 51 | 11A, 11B |
| V117.24 | mG*mA*mU*G*G*U*C*A*G*C*A*C*A*G*C*C*U*U*A*U*G*C*A*C*G* GCmC mUCG GAG*A*G*C*U*U*C*A*G*G*G*G*U*G*C*C*U*C*C*U*C*U*G* mU*mG*mA | 52 | 11A, 11B, 11C |
| V117.25 | mC*mA*mU* G*G* fC*fC*fC*fC* A*G* fC* A*G* fC*fU*fU*fC* A*G* fU*fC*fC*fC* fU*fUfUfC **dTdCdI** fUfC G*A* fU* G*G* fU*fC* A*G* fC* A* fC* A*G* fC*fC*fU*fU* A* fU* G* fC*A* mC*mG*mG | 53 | 11A, 11B, 11C |
| V117.26 | mC*mA*mU*G*G* fC*mC* fC* mC*A*G* fC* A*G* mC* fU* mU* fC* A*G* mU* fC* mC* fC* mU* fU mU fC dTdCdI mU fC G*A* mU* G*G* fU* mC* A*G* fC* A* mC*A*G* fC*mC* fU*mU* A* fU* G* fC*A* mC*mG*mG | 54 | 11A, 11B, 11C |
| V120.2 | mA*mA*mA*A*A*C*A*U*G*G*C*C*C*A*G*C*A*G*C*U*U*C*A*G*U *C*C*C*U*UUC UCG UCG*A*U*G* mG*mU*mC | 55 | 11A, 11B |
| V120.10 | mA*mA*mA*A*A*C*A*U*G*G*C*C*C*A*G*C*A*G*C*U*U*C*A*G*U *C*C*C*U*G UC  mUCG  UCG*A*U*G*mG*mU*mC | 56 | 11A, 11B |
| ADAR60-6 (WO 2018/041973) | mU*mC*mA*mG*mUmCmCmCmUmUmUmC dTdCdG mUmCmGmAmUmGmGmUmCmAmGmC*mA*mC*mA*mG | 57 | 11A, 11B |
| ADAR60-10 (WO 2018/041973) | mU*mC*mA*mG*mUmCmCmCmUmUmUmC UCl mUmCmGmAmUmGmGmUmCmAmGmC*mA*mC*mA*mG | 58 | 11A, 11B |

### Example 22: Restoration of a-1-antitrypsin measured by sandwich ELISA with cell supernatant of cells treated with constructs targeting the E342K mutation site of the human SERPINA1 transcript (Figure 11B).

Since A1AT is a secreted protein, the effect of successful RNA editing on the protein restoration can be tested by measuring the amount of A1AT secreted from cells treated with the editing constructs compared to untreated cells. This was done in the plasmid-transfected approach. The sequences of the constructs used for this example are listed in Table 13.

HeLa cells were treated as described for the plasmid-transfected approach in with 2.5 x 10⁴ cells per 24-well, 300 ng plasmid and 5 pmol construct. 48 h after construct transfection, 300 µl of the cell supernatant was removed from the cells and centrifuged at 300 x g for 5 min. The supernatant was diluted 1:30 in dilution buffer (1 x PBS + 0.05% Tween-20 + 2% bovine serum albumin (BSA)) and 100 µl were applied to a 96-well on a Greiner Microlon^{®} High Binding 96-well plate that had been coated with rabbit anti-human α-1-antitrypsin antibody (A0012, Dako Denmark ApS), washed with washing buffer (1 x PBS plus 0.05% Tween-20) and blocked with 5 % milk in 1 x PBS for 1 h. Samples were measured in triplicates and were loaded together with a human A1AT standard (SRP6312, Sigma-Aldrich) and incubated for 80 min at RT. Afterwards, the plate was washed with washing buffer and 100 µl of goat anti-human A1AT antibody conjugated to horseradish peroxidase (Biomol, A56-122P) was applied to each well at a 1:10 000 dilution in 1 x PBS, and incubated for 1h at RT. The plate was washed with washing buffer again, and 100 µl of OPD substrate (9 ml Millipore water, 1 ml Stable Peroxide Substrate Buffer 10x (34062, Thermo Scientific) and 1 x OPD Tablet (34006, Thermo Scientific)) was applied to each well and incubated for 30 min at RT in the dark. The reaction was stopped with 50 µl of 2.5 M H₂SO₄ and the OD-values were immediately read at λ = 490 nm in a Tecan Spark^{®} 10M Plate Reader. Protein concentrations were calculated from the standards, then values were normalized to those of the wildtype cell supernatants and supernatants from mutant cells transfected with a non-targeting negative control construct ("GAPDH V117.19", Seq. ID 5).

As shown in Figure 11B, the A1AT amount secreted by the treated cells mirrors the editing activities found in Example 21. The unstable construct that showed low editing activities, V117.24 (Seq. ID 52), also shows no A1AT secretion compared to the controls. However, both stable versions with the inosine nucleoside in the Central Base Triplet (V117.25, Seq. ID 53 and V117.26, Seq. ID 54) show secreted A1AT levels near half those of the wildtype positive control. Again, as already seen in Example 15/16 (Fig. 9), and Example 21, the mixture of 2'-F and 2 -O-methyl instead of only 2'-F at the pyrimidine nucleosides (V117.25 vs. 117.26) improved protein restoration.

### Example 23: Stabilization of constructs targeting the disease-causing E342K mutation of the human SERPINA1 transcript with endogenous ADAR (Figure 11C).

As discussed in Example 4, constructs that are stable against endogenous defense mechanisms, such as RNases found in serum, are preferred for the therapeutic perspective of this invention. For this, preferred embodiments used in Example 22 with (V117.25, Seq. ID 53 and V117.26, Seq. ID 54) and without (V117.24, Seq. ID 52) stabilizations by introduction of chemical modifications at pyrimidine nucleosides were tested for their stability in 10 % fetal bovine serum over the course of 7 days. Construct sequences are provided in Table 13. Results were obtained as described in Example 4.

As shown in Figure 11C and also for the previous targets, the modification of pyrimidine nucleosides within the construct, as for V117.25 and V117.26, allows the construct to withstand in 10% FBS for over 7 days, while the unstable construct V117.24 is immediately degraded. Overall, Examples 21 to 23 show the promising properties of the invention with regard to therapeutic application.

To discern the current invention from prior arts, two oligonucleotides disclosed in WO 2018/041973, ADAR60-6 (here Seq. ID 57) and ADAR60-10 (here Seq. ID 58), were chosen to compare editing activities and protein restoration under the same conditions. Both oligonucleotides were chosen for their relatively close similarity in terms of modification types, density and positioning. Both oligonucleotides were composed of 31 nucleotides, in which the equivalent of their Central Base Triplets was placed symmetrically within the overall structure, just as in a preferred embodiment of the current invention, v117.19 (Figure 3B). The oligonucleotide backbones of the prior art comprised of 4 consecutive phosphorothioate linkages at both termini, thus contained fewer phosphorothioate linkages as presented in the embodiments V117.25 (Seq. ID 53) and V117.26 (Seq. ID 54) of the current example, which contained 25 consecutive phosphorothioate linkages at each termini. Finally, ADAR60-6 possessed a Central Base Triplet fully comprised of DNA nucleosides, whereas ADAR60-10 possessed a Central Base Triplet containing an inosine nucleoside 3'-adjacent to the base opposite of the targeted adenosine. Combined, the two oligonucleotides ADAR60-6 (Seq. ID 57) and ADAR60-10 (Seq. ID 58) showed similar modifications as the preferred embodiments V117.25 (Seq. ID 53) and V117.26 (Seq. ID 54) presented in the current invention and were thus deemed suitable for a comparison to the prior art. However, they differ from the present invention in the length of the oligonucleotide, the low phosphorothioate content and the usage of large blocks of 2 -O-methlyated bases. Their exact sequences are disclosed in Table 13.

As shown in Figure 11A), although ADAR60-6 and ADAR60-10 were similar compared to the embodiments presented in Examples 21-23 as described in the previous paragraph, no traceable editing activity was detected for either oligonucleotide under the tested conditions (see Example 21 and Figure 11A)). Furthermore, ADAR60-6 and ADAR60-10 were unable to restore the A1AT protein, as shown via A1AT sandwich ELISA (see Example 22 and Figure 11B)), whereas the leading construct targeting the E342K mutated SERPINA1 target of the current invention, V117.26 (Seq. ID 54), restored an average of 42% of the wildtype A1AT control. The prior art solution was unable to achieve notable editing with endogenous ADAR. This data fits to the data presented in the original patent WO 2018/041973 (Fig. 3), which requires overexpression of ADAR2 and a highly artificial conduction of the editing experiment in cell lysate to achieve detectable editing.

Overall, the data demonstrates that our current invention is superior to the prior art in terms of RNA editing yields and protein restoration with endogenous ADARs inside the living cell.

### Example 24: Editing of the disease-causing G2019S mutation in the human LRRK2 transcript with endogenous ADAR (Figure 12).

As a key kinase in the nervous system, mutations in the LRRK2 gene are associated with neurodegenerative diseases. One of the pathogenic mutations, G2019S, increases the risk of developing Parkinson's disease. The target adenosine underlying the G2019S mutation is in a 5'-CAG context. Thus, it is a suitable target for the current invention. Construct sequences are provided in Table 14.

5x 10⁴ HeLa cells were seeded in a 24-well plate. After 24 h cells were forward transfected with a plasmid containing the human LRRK2 cDNA bearing the G2019S mutation. 300 ng plasmid and 0.9 µl FuGENE^{®} 6 (Promega) were each diluted in 50 µl Opti-MEM and incubated for 5 min, then combined and incubated for an additional 20 min. The medium was changed, and the transfection mix evenly distributed into one well. 24 h after plasmid transfection, cells were forward transfected with 25 pmol ASO/well and 1.5 µl/well Lipofectamine RNAiMAX Reagent (ThermoFisher Scientific). After 24 h, cells were harvested for RNA isolation and sequencing.

As shown in Figure 12, the editing yields obtained with the constructs without chemically modified pyrimidine nucleosides (V117.19, Seq. ID 59 and V120.2, Seq. ID 61) are very high, ranging between 60 and 80%. Upon the introduction of the chemically modified pyrimidine nucleosides, namely V117.20 (Seq. ID 60) and V120.3 (Seq. ID 62), editing activity of the constructs decreases by about half. This decrease in editing activity upon introduction of chemical modifications on the pyrimidine nucleosides is a trend that has already been noticed in previous examples. Together with the SERPINA1 target, is can be proved that the previous invention is also useful to target adenosines in a context lesser preferred by ADAR, such as for 5'-CAG as shown here.

**Table 14: Construct sequences and modifications from Example 24**

| mN = 2'-O-methyl, fN = 2'-fluoro, N = 2'OH (ribo), **dN** = 2'H (deoxyribo), * = phosphorothioate linkage | | | |
|---|---|---|---|
| Construct Name | Construct Sequence (5' to 3' direction) | Seq. ID | Figure No. |
| V117.19 | mC*mC*mC*C*A*U*U*C*U*A*C*A*G*C*A*G*U*A*C*U*G*A*G*C*A*AU G  CCdI UA G*U*C*A*G*C*A*A*U*C*U*U*U*G* C*A*A*U*G*A*U*G*G*mC*mA*mG | 59 | 12 |
| V117.20 | mC*mC*mC*fC*A*mU*fU*mC*fU*A*mC*A*G*fC*A*G*mU*A*fC*mU*G* A*G*fC*A*AmUG dCdCdI fUAG*mU*fC*A*G*mC*A*A*fU*mC*fU*mU*fU*G*mC*A*A*fU*G*A*mU* G*G*mC*mA*mG | 60 | 12 |
| V120.2 | mU*mU*mU*A*U*C*C*C*A*U*U*C*U*A*C*A*G*C*A*G*U*A*C*U*G*A *G*C*A*AUG CCdI UAG*U*C*A*mG*mC*mA | 61 | 12 |
| V120.3 | mU*mU*mU*A*fU*mC*fC*mC*fC*A*mU*fU*mC*fU*A*mC*A*G*fC*A*G* mU*A*fC*mU*G*A*G*fC*A*AmUG dCdCdI fUAG*mU*fC*A*mG*mC*mA | 62 | 12 |

## Claims

1. A chemically modified oligoribonucleotide for use in site-directed A-to-I editing of a target RNA inside a cell with endogenous ADAR, comprising a sequence with a length from 25 to 80 nucleotides, capable of binding to a target sequence in the target RNA, comprising a Central Base Triplet of 3 nucleotides with the central nucleotide opposite to the target adenosine in the target RNA which is to be edited to an inosine,
**characterized in that**
a)
at least 90% of the pyrimidine nucleosides outside the Central Base Triplet are chemically modified, either at the 2' position of the sugar moiety, or are deoxyribonucleosides, or a combination thereof,
b) no more than 6 consecutive nucleosides are chemically modified with 2 -O-methyl at the 2' position of the sugar moiety,
c) at least two of the three nucleosides of the Central Base Triplet are chemically modified at the 2' position of the sugar moiety, or are deoxyribonucleosides, or a combination thereof.

2. A chemically modified oligoribonucleotide according to claim 1, wherein the nucleoside opposite to the target adenosine to be edited in the target RNA carries an N-heterocyclic base, more preferably a pyridine or pyrimidine base, preferably selected from the group of cytosine, uracil, thymine or 5-methylcytosine, most preferably a cytosine.

3. A chemically modified oligoribonucleotide according to any of the preceding claims, wherein at least 30%, preferably at least 40%, more preferably at least 50%, most preferably at least 60% of the linkages between the nucleosides are phosphorothioate linkages.

4. A chemically modified oligoribonucleotide according to any of the preceding claims, wherein the oligoribonucleotide comprises at least one nucleotide block of consecutive phosphorothioate linkages between the nucleosides with at least 5, preferably at least 10, more preferably at least 15, most preferably at least 20 phosphorothioate linkages.

5. A chemically modified oligoribonucleotide according to any of the preceding claims, wherein the chemical modifications of the pyrimidine nucleosides outside the Central Base Triplet constitute a combination of 2'-fluoro and 2'-O-methyl substituents, whereby at least 20%, preferably 30-70%, more preferably 40-60% of the chemical modifications are 2 -O-methyl substituents.

6. A chemically modified oligoribonucleotide according to any of the preceding claims, wherein no more than 6, more preferably no more than 5, most preferably no more than 4 consecutive nucleosides comprise a 2 -O-methyl modification.

7. A chemically modified oligoribonucleotide according to any of the preceding claims, wherein 100% of the pyrimidines are modified, either at the 2' position of the sugar moiety, or are deoxyribonucleosides, or a combination thereof.

8. A chemically modified oligoribonucleotide according to any of the preceding claims, wherein each of the three nucleosides in the Central Base Triplet is a deoxyribonucleotide.

9. A chemically modified oligoribonucleotide according to any of the preceding claims, wherein the nucleoside at the 3' position of the Central Base Triplet consists of an inosine, or a derivative thereof, when targeting an adenosine in a 5'-CAN sequence context (N = G, A, U, or C) so that the inosine in the oligoribonucleotide is opposite the cytosine base in the target (m)RNA.

10. A chemically modified oligoribonucleotide according to any of the preceding claims, wherein a hairpin-loop structured ADAR recruiting moiety is absent.

11. A chemically modified oligoribonucleotide according to any of the preceding claims, wherein the two nucleotide sequences flanking the Central Base Triplet comprise the same length.

12. A chemically modified oligoribonucleotide according to any of the preceding claims, wherein the two nucleotide sequences flanking the Central Base Triplet comprise different lengths, whereby the sequence at the 3' terminus amounts to at least 4 nucleotides, preferably at least 9 nucleotides, and the sequence at the 5' terminus amounts to at least 19 nucleotides, preferably at least 28 nucleotides, more preferably at least 33 nucleotides.

13. A chemically modified oligoribonucleotide according to any of the preceding claims, wherein 3' and 5' termini are modified each with a block of three consecutive 2 -O-methyl phosphorothioate nucleotides or three 2-O-methoxyethyl (2' MOE) phosphorothioate nucleotides.

14. A chemically modified oligonucleotide according to any of the preceding claims wherein both termini at the 3' and 5' end contain at least 5 consecutive phosphorothioate linkages.

15. Medicament for treatment or prevention of a human disorder or disease, preferably of a genetic disorder or disease, **characterized in that** it comprises at least one chemically modified oligoribonucleotide according to any of the preceding claims.

16. Chemically modified oligoribonucleotide according to any of claims 1-14 for use in the treatment or the prevention of alpha1-antitrypsin deficiency by targeting the common E342K mutation in human SERPINA1, or for treatment or prevention of neurodegenerative diseases by targeting the common S2019G mutation in human LRRK2, or for treatment or prevention of mucopolysaccharidosis by targeting the common W402X mutation in human IDUA, or for treatment and prevention of Retinitis pigmentosa by targeting the V685M mutation in human PDE6A, or for treatment and prevention of Zellweger spectrum disorder by targeting the G843D mutation in the human PEX1 gene, or for treatment or prevention of Stargardt disease or age-related macular degeneration by targeting the G1961E mutation in human ABCA4, or for treatment or prevention of retinal diseases like Retinitis pigmentosa or Leber's congenital amaurosis by targeting the C948Y mutation in human CRB1.
